(19) Europäisches Patentamt
European Patent Office
Office européen des brevets

(11) Publication number: **0 144 098**
**A1**

# (12) EUROPEAN PATENT APPLICATION

(21) Application number: 84201498.7

(22) Date of filing: 16.10.84

(51) Int. Cl.⁴: C 07 D 413/04
C 07 D 498/04, A 01 N 43/50
A 01 N 43/80

(30) Priority: 26.10.83 JP 201558/83

(43) Date of publication of application:
12.06.85 Bulletin 85/24

(84) Designated Contracting States:
CH DE FR LI

(71) Applicant: SHIONOGI & CO., LTD.
12, Dosho-machi 3-chome Higashi-ku
Osaka 541(JP)

(72) Inventor: Sumitomo, Shinzaburo
35-18, Iwazono-cho
Ashiya-shi Hyogo(JP)

(72) Inventor: Ishizuka, Ichiro
4-5-8, Tokiwadai Toyono-cho
Toyono-gun Osaka(JP)

(72) Inventor: Ide, Kinya
170, Hirai-cho
Kusatsu-shi Shiga(JP)

(72) Inventor: Ueyama, Yoshito
963-20, Kamigasa-cho
Kusatsu-shi(JP)

(74) Representative: Hranitzky, Wilhelm Max et al,
NOVAPAT - CABINET CHEREAU 5, Place du Molard
CH-1204 Genève(CH)

## (54) Cyclic ureas of isoxazole.

(57) Cyclic ureas of isoxazole (I) which show potent herbicidal activity.

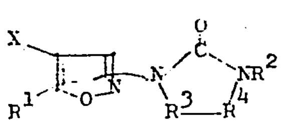
(I)

(wherein $R^1$ is $C_1$-$C_5$ alkyl; $R^2$ is hydrogen, $C_1$-$C_5$ alkyl, or $C_2$-$C_5$ alkenyl; $-R^3$-$R^4-$ is

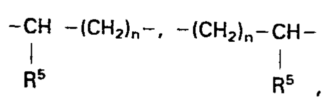

$-CO-CH_2-$, $-CO-CO-$, $-CH(OY)-CH(OY)-$, $-CH(OY)-CH_2-$, 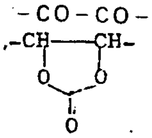

$-CH(OZ)-CO-$, or $-CO-CH(OZ)$; $R^5$ is hydrogen or $C_1$-$C_3$ alkyl; n is 1 or 2; X is hydrogen or halogen; Y is $C_1$-$C_5$ alkyl, $C_2$-$C_5$ alkenyl, $C_2$-$C_5$ alkoxycarbonyl, or N-($C_1$-$C_3$ alkyl)-carbamoyl; and Z is hydrogen, $C_2$-$C_5$ alkanoyl, $C_2$-$C_5$ alkoxycarbonyl, N-($C_1$-$C_3$ alkyl)carbamoyl, or benzoyl which may be substituted).

EP 0 144 098 A1

## BACKGROUND OF THE INVENTION

### 1. FIELD OF THE INVENTION

The present invention relates to cyclic ureas of isoxazole which show potent herbicidal activity.

### 2. PRIOR ART

As compounds analogous to isoxazolyl imidazolidinone, 3-[3(5)-isoxazolyl]-4-hydroxy-2-imidazolidinones and the like are disclosed in U.S. Pat. No. 4,268,679 and U.K. Pat. No. 2.079.283.

## BRIEF SUMMARY OF THE INVENTION

The present invention relates to cyclic ureas of isoxazole. More particularly, it relates to the compounds of the formula (I):

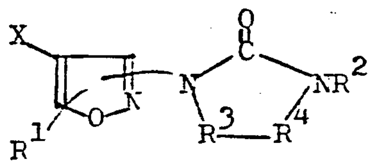

(I)

(wherein $R^1$ is $C_1$-$C_5$ alkyl; $R^2$ is hydrogen, $C_1$-$C_5$ alkyl, or $C_2$-$C_5$ alkenyl; $-R^3—R^4-$ is $-\underset{R^5}{CH}-(CH_2)_n-$, $-(CH_2)_n-\underset{R^5}{CH}-$, $-CO-CH_2-$, $-CO-CO-$, $-CH(OY)-CH(OY)-$, $-CH(OY)-CH_2-$, 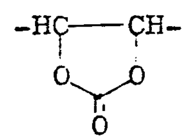 $-CH(OZ)-CO-$, or $-CO-CH(OZ)-$; $R^5$ is hydrogen or $C_1$-$C_3$ alkyl; n is 1 or 2; X is hydrogen or halogen; Y is $C_1$-$C_5$ alkyl, $C_2$-$C_5$ alkenyl, $C_2$-$C_5$ alkoxycarbonyl, or N-($C_1$-$C_3$ alkyl)-carbamoyl; and Z is hydrogen, $C_2$-$C_5$ alkanoyl, $C_2$-$C_5$ alkoxycarbonyl, N-($C_1$-$C_3$ alkyl)carbamoyl, or benzoyl which may be substituted). Furthermore, herbicidal compositions containing one or more species of said compounds (I) are provided. The compounds (I) can be prepared by

(A) cyclizing a compound of the formula (II):

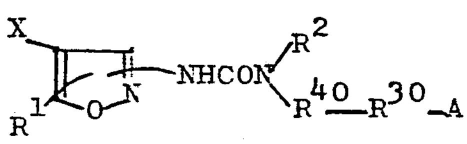

(II)

with a condensing agent to give a compound of the formula (Ia):

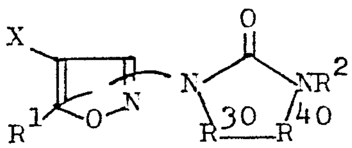

(Ia)

(B) reacting a compound of the formula (III):

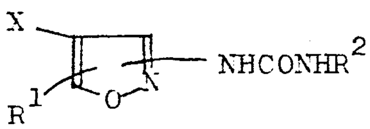

(III)

with glyoxylic acid or an oxalyl halide to give a compound of the formula (Ib):

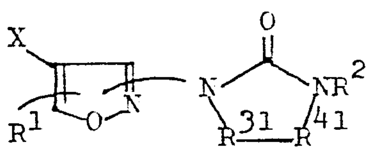

(Ib)

and if required, reducing the compound of the formula (Ib) wherein $-R^{31}-R^{41}-$ is -CO-CO- to yield a compound of the formula (Ic):

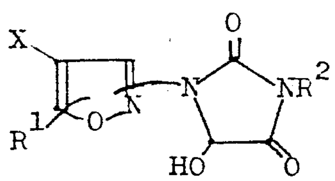

(Ic)

(C) subjecting a compound of the formula (IV):

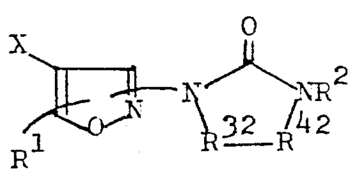

(IV)

to etherification, acylation, or amidation to give a compound of the formula (Id):

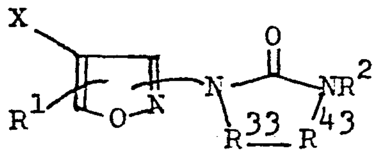

(Id)

or (D) reacting a compound of the formula (V):

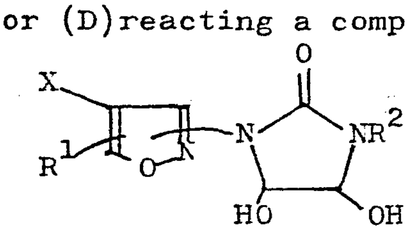

(V)

with a carbonylating agent to give a compound of the formula (Ie):

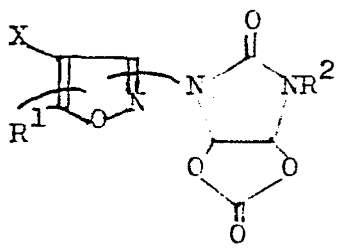

(Ie)

(in all of the above formulae, $R^1$, $R^2$, and X each has the same significance as defined above: $-R^{30}-R^{40}-$ is $-\underset{R^5}{CH}-(CH_2)_n-$, $-(CH_2)_n-\underset{R^5}{CH}-$ , or $-CO-CH_2-$; $R^5$ is hydrogen or $C_1$-$C_3$ alkyl; n is 1 or 2; A is a reactive residue such as halogen, hydroxy, alkoxy, or acyloxy; $-R^{31}-R^{41}-$ is $-CO-CH(OH)-$ or $-CO-CO-$; $-R^{32}-R^{42}-$ is $-CH(OH)-CH_2-$, $-CH(OH)-CH(OH)-$, $-CH(OH)-CO-$, or $-CO-CH(OH)-$; $-R^{33}-R^{43}-$ is $-CH(OY)-CH_2-$, $-CH(OY)-CH(OY)-$, $-CH(OZ^1)-CO-$, or $-CO-CH(OZ^1)$; Y is $C_1$-$C_5$ alkyl, $C_2$-$C_5$ alkenyl, $C_2$-$C_5$ alkoxycarbonyl, or N-($C_1$-$C_3$ alkyl)carbamoyl; and $Z^1$ is $C_2$-$C_5$ alkanoyl, $C_2$-$C_5$ alkoxycarbonyl, N-($C_1$-$C_3$ alkyl)-carbamoyl, or benzoyl which may be substituted.)

In the definition of $-R^3-R^4-$ ($-R^{30}-R^{40}-$, $R^{31}-R^{41}-$, $-R^{32}-R^{42}-$, $-R^{33}-R^{43}-$), $R^3$ is bound to the nitrogen atom which is substituted by an isoxazolyl group and $R^4$ is bound to the nitrogen atom which is substituted by $R^2$.

The present compounds (I) are useful as herbicides.

## DETAILED DESCRIPTION OF THE INVENTION

The present invention relates to cyclic ureas of isoxazole. More particularly, it relates to the compounds of the formula (I);

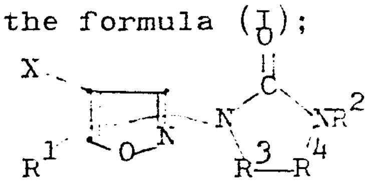

(I)

(wherein $R^1$ is $C_1$-$C_5$ alkyl; $R^2$ is hydrogen, $C_1$-$C_5$ alkyl, or $C_2$-$C_5$ alkenyl; $-R^3-R^4-$ is $-CH(R^5)-(CH_2)_n-$, $-(CH_2)_n-CH(R^5)-$,

$-CO-CH_2-$, $-CO-CO-$, $-CH(OY)-CH(OY)-$, $-CH(OY)-CH_2-$, 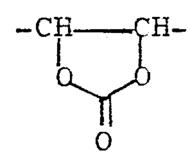,

$-CH(OZ)-CO-$, or $-CO-CH(OZ)-$; $R^5$ is hydrogen or $C_1$-$C_3$ alkyl; n is 1 or 2; X is hydrogen or halogen; Y is $C_1$-$C_5$ alkyl, $C_2$-$C_5$ alkenyl, $C_2$-$C_5$ alkoxycarbonyl, or N-($C_1$-$C_3$ alkyl)-carbamoyl; and Z is hydrogen, $C_2$-$C_5$ alkanoyl, $C_2$-$C_5$ alkoxycarbonyl, N-($C_1$-$C_3$ alkyl)carbamoyl, or benzoyl which may be substituted.)

The compounds (I) may be prepared by Routes 1 to 7

according to each reaction sequence described below.

Route 1

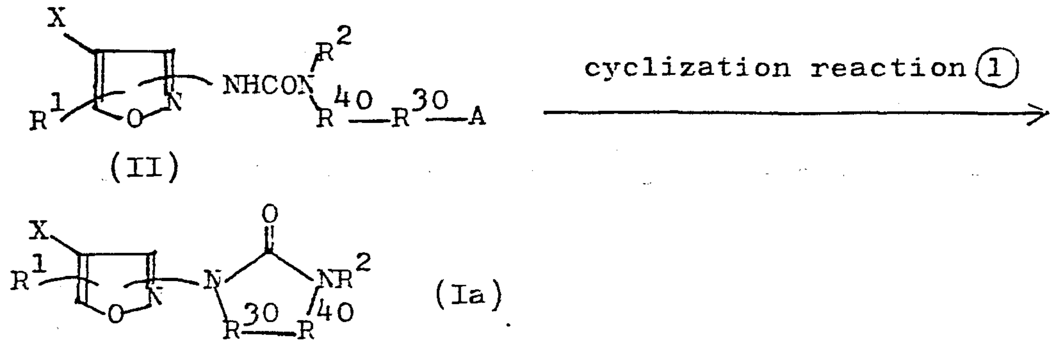


(II)

(Ia)

(wherein $R^1$, $R^2$, and X each has the same meaning as defined above; $-R^{30}-R^{40}-$ is $-\underset{R^5}{CH}-(CH_2)_n-$ , $-(CH_2)_n-\underset{R^5}{CH}-$ , or $-CO-CH_2-$; $R^5$ is hydrogen or $C_1$-$C_3$ alkyl; n is 1 or 2; and A is a reactive residue such as halogen (e.g. chlorine, bromine, iodine), hydroxy, alkoxy, or acyloxy.)

The reaction may be carried out at a temperature of about 0 to about 150°C, preferably 15 to 100°C in an appropriate solvent in the presence of a condensing agent. Representatives of the solvents are hydrocarbons (e.g. benzene, toluene), halogenohydrocarbons (e.g. methylene chloride, 1,2-dichloroethane, chloroform, carbon tetrachloride), dimethylformamide, dimethylsulfoxide, alkanols (e.g. methanol, ethanol, isopropanol), and the like. The condensing agent may be selected suitably from the group consisting of inorganic bases such as alkali metal hydroxide (e.g. lithium hydroxide, potassium hydroxide, sodium hydroxide),

alkali metal carbonate (e.g. potassium carbonate, sodium carbonate), and alkali metal hydride (e.g. sodium hydride), organic bases (e.g. pyridine, triethylamine), DCC (dicyclohexylcarbodiimide), toluenesulfonic acid, and the like.

The starting materials (II) are readily prepared from the corresponding phenyl isoxazolyl carbamates (X) or isoxazolyl isocyanate (XI).

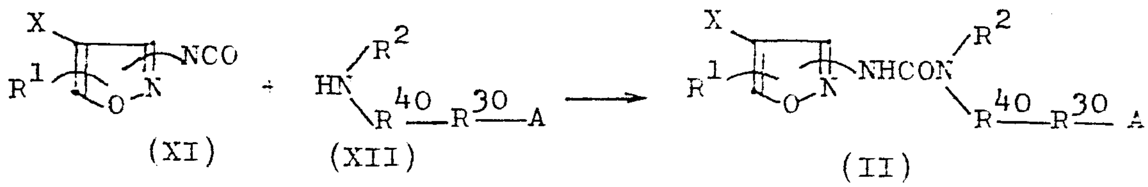

(XI) (XII) (II)

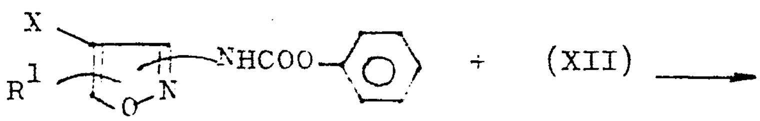

(XII) (II)

(X): [U.K. Pat. No. 2,079.283]

Route 2

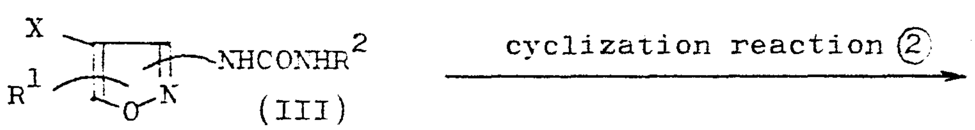


(III)

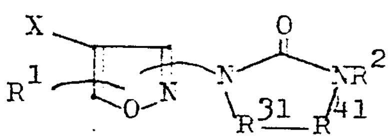

(Ib)

(wherein $R^1$, $R^2$, and X each has the same meaning as defined above; and $-R^{31}—R^{41}-$ is -CO-CH(OH)- or -CO-CO-.)

This reaction may be carried out, using glyoxylic acid or an oxalyl halide (e.g. oxalyl chloride, oxalyl bromide) in a suitable solvent, if required, in the presence of an appropriate condensing agent at a temperature of about 0 to about 150°C, preferably 50 to 100°C.

The solvent and condensing agent used in this reaction may be selected suitably from those described in Route 1.

The starting material (III) is prepared according to the procedures described in U.S. Pat. Nos. 4,028,376 and 4,062,861.

Route 3

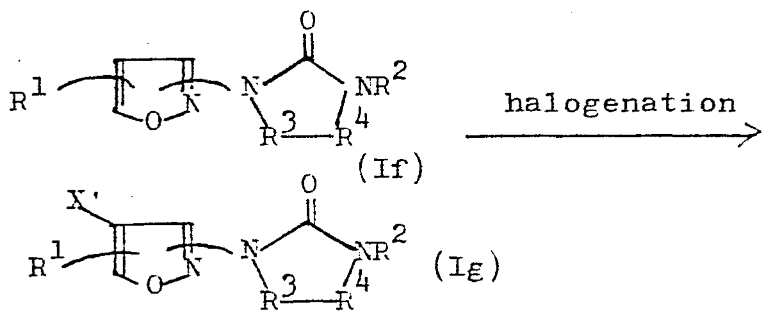


(If)

(Ig)

(wherein $R^1$, $R^2$, and $-R^3-R^4-$ each has the same significance as defined above; and X' is halogen.) This reaction can be conducted in a conventional manner for halogenating the benzene ring of aromatic compounds and may generally be achieved with a molecular halogen or sulfuryl chloride in a suitable solvent at a temperature of about 0 to about 100°C, preferably 10 to 60°C. The suitable solvent may be selected from halogenohydrocarbons (e.g. chloroform, methylene chloride, 1,2-dichloroethane, carbon tetrachloride) and acetic acid in compliance with the purpose of the reaction.

Route 4

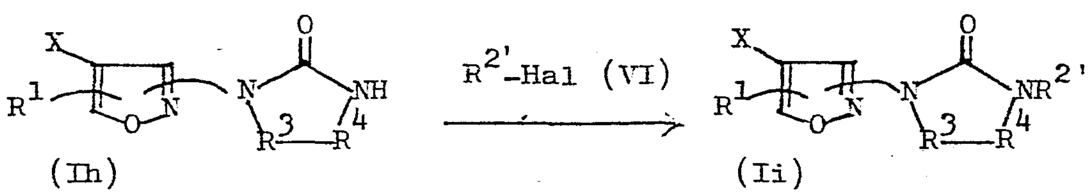


(Ih) (Ii)

(wherein $R^1$, $-R^3-R^4-$, and X each has the same meaning as defined above, $R^{2'}$ is $C_1$-$C_5$ alkyl or $C_2$-$C_5$ alkenyl; and Hal is halogen.)

In this route the starting material (Ih) is allowed to react with alkyl halide (e.g. methyl iodide, butyl bromide, isopropyl chloride) or alkenyl halide (e.g. allyl bromide, 3-butenyl iodide).

This reaction may be carried out at a temperature of about -30 to about 150°C, preferably 0 to 100°C in the presence of an inorganic base (e.g. sodium hydride, sodium amide, sodium hydroxide) or an alkali metal alkoxide (e.g. potassium t-butoxide, sodium methoxide) in a suitable solvent (e.g. alkanol such as methanol, ethanol, or isopropanol; dimethylsulfoxide).

Route 5

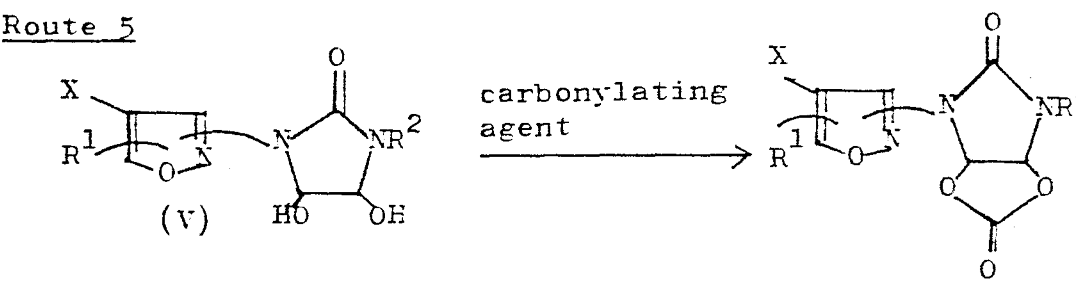


(V) (Ie)

(wherein $R^1$, $R^2$, and X each has the same meaning as defined above.)

This reaction may be carried out at a temperature of about 0 to about 100°C, preferably 10 to 30°C in the presence of a carbonylating agent (e.g. 1,1'-carbonyldiimidazole, phosgene) in a suitable solvent (e.g. chloroform, dimethylformamide, methylene chloride, benzene).

The starting material (V) can be prepared by the procedure described in U.S. Pat. No. 4,268,679.

Route 6

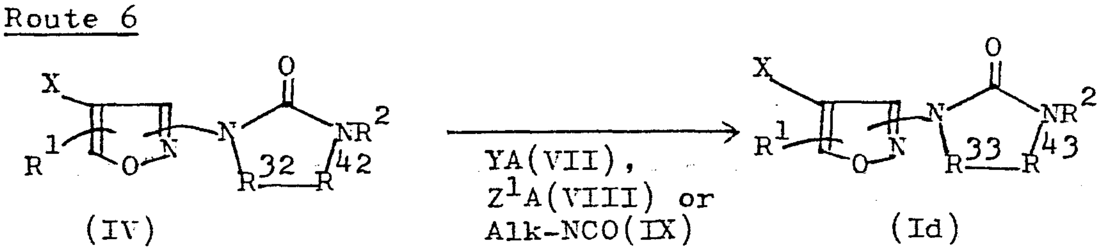


(wherein A, $R^1$, $R^2$, and X each has the same meaning as defined above; $-R^{32}$—$R^{42}-$ is $-CH(OH)-CH_2-$, $-CH(OH)-CH(OH)-$, $-CH(OH)-CO-$ , or $-CO-CH(OH)-$; $-R^{33}$—$R^{43}-$ is $-CH(OY)-CH_2-$, $-CH(OY)-CH(OY)-$, $-CH(OZ^1)-CO-$, or $-CO-CH(OZ^1)-$; Y is $C_1-C_5$ alkyl, $C_2-C_5$ alkenyl, $C_2-C_5$ alkoxycarbonyl, or N-($C_1-C_3$-alkyl)carbamoyl; and $Z^1$ is $C_2-C_5$ alkanoyl, $C_2-C_5$ alkoxy-carbonyl, N-($C_1-C_3$ alkyl)carbamoyl, or benzoyl which may be substituted (wherein the substituent is halogen, $C_1-C_5$ alkyl, or $C_1-C_4$ alkoxy))

In this route the alcoholic hydroxy group is converted into an ether, acyloxy, or carbamoyloxy, and the reactions may be carried out in a conventional manner respectively. The etherification is achieved in the presence of a base (e.g. sodium methoxide, potassium t-butoxide) with an etherifying agent (VII) such as alkyl halide (e.g. methyl bromide, methyl iodide, ethyl iodide) or alkenyl halide. The acylation is achieved with an acylating agent (VIII) (e.g. acetyl chloride, acetic anhydride, benzoyl chloride, ethyl

chloroformate, methyl chloroformate), and optionally in the presence of an organic base (e.g. triethylamine, pyridine). The amide formation is carried out on reaction with an alkyl isocyanate (IX), if required, in the presense of a base. Each of these reactions may be carried out at a temperature of about -100 to about 80°C, preferably -78 to 60°C, optionally in a suitable solvent (e.g. benzene, toluene, chloroform, dimethylformamide, tetrahydrofuran).

The starting material (IV) in which $-R^{32}-R^{42}$ is $-CH(OH)-CH_2-$ can be prepared according to the procedure disclosed in U.S. Pat. No. 4,268,679. The starting material (IV) wherein $-R^{32}-R^{42}-$ is $-CO-CH(OH)-$ can be prepared in the Route 2. The compound IV $[-R^{32}-R^{42} = -CH(OH)-CO-]$ may be prepared in the Route 7.

Route 7

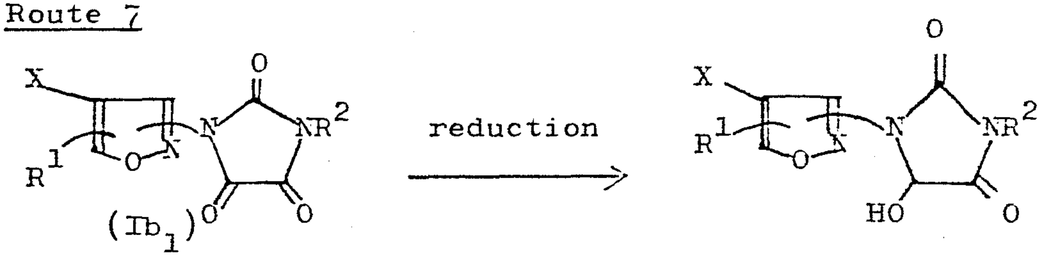


(Ib$_1$) (Ic)

(wherein $R^1$, $R^2$, and X each has the same meaning as defined above.)

The reaction may be carried out with sodium borohydride at a temperature of about 0 to about 100°C, preferably 15 to 50°C in a suitable solvent (e.g. methanol, N,N-dimethylformamide, tetrahydrofuran).

The starting material (Ib$_1$) can be prepared by way of

Route 2 mentioned above.

The meanings of the terms used in the above definition are shown below: the $C_1$-$C_5$ alkyl includes methyl, ethyl, n-propyl, isopropyl, n-butyl, isobutyl, sec-butyl, tert-butyl, n-pentyl, isopentyl, sec-pentyl, neo-pentyl, tert-pentyl, and the like; the $C_2$-$C_5$ alkenyl includes vinyl, allyl, 1-propenyl, isopropenyl, 3-butenyl, 1,3-butadienyl, 2-pentenyl, and the like; the $C_1$-$C_3$ alkyl means methyl, ethyl n-propyl, isopropyl, and the like; examples of halogen are fluorine, chlorine, bromine, iodine, and the like; the $C_2$-$C_5$ alkoxycarbonyl means carbonyl which is substituted by $C_1$-$C_4$ alkoxy, and the $C_1$-$C_4$ alkoxy is exemprified by methoxy, ethoxy, n-propoxy, isopropoxy, n-butoxy, isobutoxy, sec-butoxy, tert-butoxy, and the like;

in the N-($C_1$-$C_3$ alkyl)carbamoyl, the $C_1$-$C_3$ alkyl has the same meaning as explained above: the $C_2$-$C_5$ alkanoyl includes acetyl, propionyl, butyryl, isobutyryl, valeryl, isovaleryl, pivaloyl, and the like; in the benzoyl which may be substituted, examples of substituents are halogen, $C_1$-$C_5$ alkyl, $C_1$-$C_4$ alkoxy, and the like; the $C_1$-$C_5$ alkyl and the $C_1$-$C_4$ alkoxy each has the same significance as exemplified above; the alkoxy means $C_1$-$C_4$ alkoxy; and the acyloxy means benzoyloxy, alkoxycarbonyloxy, and alkanoyloxy, and in the alkanoyloxy, the alkanoyl means $C_1$-$C_5$ alkanoyl, for example formyl and the $C_2$-$C_5$ alkanoyl explained above.

Presently preferred and practical embodiments of the present invention are illustratively shown in the following examples. The abbreviations used in Examples and Referential Examples have the following meanings:

Me (methyl), i-Pr (isopropyl), t-Bu (t-butyl),

Ph (phenyl), Al (allyl), Ac (acetyl).

Example 1

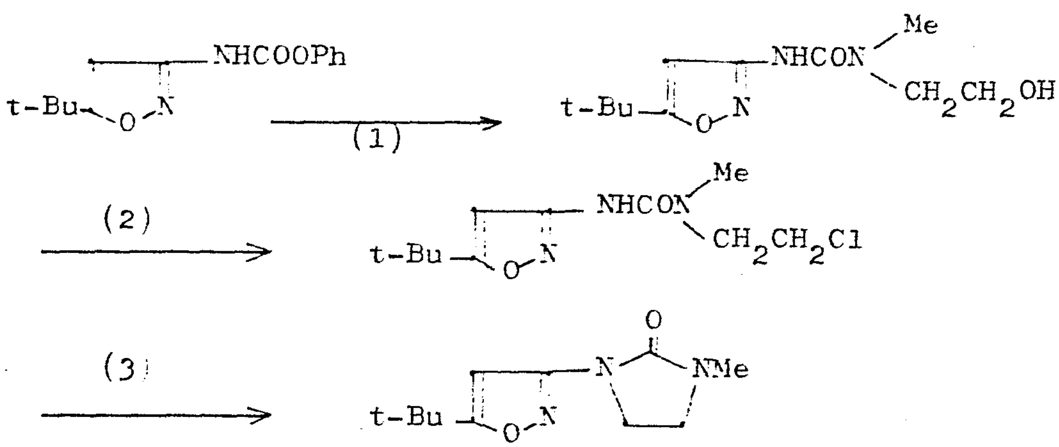


(1) To a solution of 5.20 g of phenyl 5-t-butylisoxazole-3-carbamate in 40 ml of methylene chloride is added 4.50 g of N-methyl-2-ethanolamine and the mixture is stirred at room temperature for three hours. The solvent is removed by evaporation and the residue is purified by column chromatography on silica gel to give 4.20 g (yield: 87.0 %) of the objective compound as crystals. This is recrystallized from benzene-cyclohexane to give 1-(2-hydroxyethyl)-1-methyl-3-(5-t-butyl-3-isoxazolyl)urea as colorless needles, m.p. 99.0 - 100°C.

(2) To 9.65 g of the compound prepared in the above step (1) are added 70 ml of dry benzene and 5.71 g of thionyl chloride, and the mixture is refluxed for half an hour and and then concentrated under reduced pressure. The residue is purified by chromatography on silica gel to give 9.49 g (yield: 91.3 %) of the compound as crystals. This is recrystallized from cyclohexane to give 1-(2-chloroethyl)-1-methyl-3-(5-t-butyl-3-isoxazolyl)urea as colorless prisms melting at 109.5 - 111.5°C.

(3) To 5.97 g of the compound prepared in the above step (2) is is added 25.81 g of 15 % aqueous potassium hydroxide and the mixture is stirred at room temperature for an hour. The reaction mixture is extracted with methylene chloride, and the organic layer is dried over anhydrous sodium sulfate and concentrated under reduced pressure. The residue is purified by chromatography on silica gel to give 5.06 g (yi d: 98.5 %) of the objective compound as crystals. This is recrystallized from cyclohexane to give 3-(5-t-butyl-3-isoxazolyl)-1-methyl-2-imidazolidinone as colorless prisms , m.p. 84.0 - 85.0°C.

Examples 2 - 5

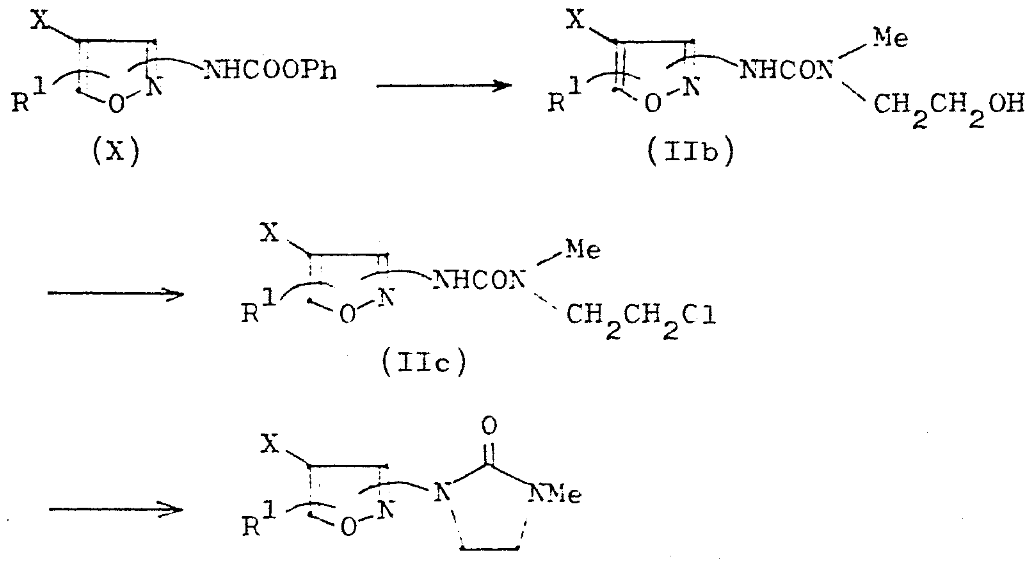

(X) (IIb) (IIc) (Ia)

The material (X) is converted into the objective compound (Ia) through the intermediates (IIb) and (IIc) under the same reaction conditions as in each step of Example 1. The results are illustrated in the following Table 1.

Table 1

| Example Nos. | IIb | | | | | II c | | | I a | | |
|---|---|---|---|---|---|---|---|---|---|---|---|
| | $R^1$ | X | Amount of (X) used (mol) | Yield (g / %) | m.p.(°C) Appearance | Amount of IIb used (mol) | Yield (g / %) | m.p.(°C) Appearance | Amount of IIc used (mol) | Yield (g / %) | m.p.(°C) Appearance |
| 2 | 5-t-Bu | Cl | 0.024 | 5.82<br>82.7 | 160.0-160.5<br>colorless plates | 0.015 | 3.01<br>66.0 | 136.0-139.0<br>colorless needles | 0.004 | 1.02<br>99.4 | 70.0-71.5<br>colorless prisms |
| 3 | 5-i-Pr | H | 0.010 | 2.02<br>88.9 | 77.0-78.0<br>colorless needles | 0.023 | 4.83<br>85.5 | 128.0-129.0<br>colorless needles | 0.010 | 2.06<br>98.4 | 75.0-76.0<br>colorless prisms |
| 4 | 3-t-Bu | H | 0.050 | 10.84<br>89.9 | 93.0-95.0<br>colorless prisms | 0.023 | 5.23<br>87.5 | 115.0-116.0<br>colorless needles | 0.010 | 2.12<br>94.8 | 146.0-147.0<br>colorless needles |
| 5 | 3-i-Pr | H | 0.050 | 8.84<br>77.7 | 101.0-102.0<br>colorless prisms | 0.023 | 4.03<br>71.3 | 99.0-101.0<br>colorless plates | 0.010 | 1.99<br>95.1 | 116.5-117.5<br>colorless plates |

Example 6

[Production of 3-(5-t-butyl-4-chloro-3-isoxazolyl)-1-methyl-2-imidazolidinone]

To 0.89 g of 3-(5-t-butyl-3-isoxazolyl)-1-methyl-2-imidazolidinone are added 4 ml of dry methylene chloride and 1.08 g of sulfuryl chloride and the mixture is refluxed for two hours. The reaction mixture is concentrated in vacuo and then purified by chromatography on silica gel to give 0.65 g (yield: 63.4 %) of the objective compound, m.p. 67.0 - 70.0°C.

Example 7

[Production of 3-(3-t-butyl-4-bromo-5-isoxazolyl)-1-methyl-2-imidazolidinone]

To a solution of 0.67 g of 3-(3-t-butyl-5-isoxazolyl)-1-methyl-2-imidazolidinone and 0.30 g of anhydrous sodium acetate in 6 ml of glacial acetic acid is dropwise added 0.57 g of bromine under ice-cooling, and the mixture is stirred at room temperature for 1.5 hours. The reaction mixture is quenched with ice water, adjusted to pH 5 with aqueous sodium hydroxide and extracted with methylene chloride. The organic layer is washed with water, dried and then concentrated in vacuo. The residue is purified by chromatography on silica gel to give 0.75 g (yield: 82.7 %) of the objective compound. This is recrystallized from cyclohexane-benzene to give anhydrous pillars,

m.p.: 122.5 - 123.5°C.

Example 8

[Production of 1-allyl-3-(5-t-butyl-3-isoxazolyl)-2-imidazolidinone]

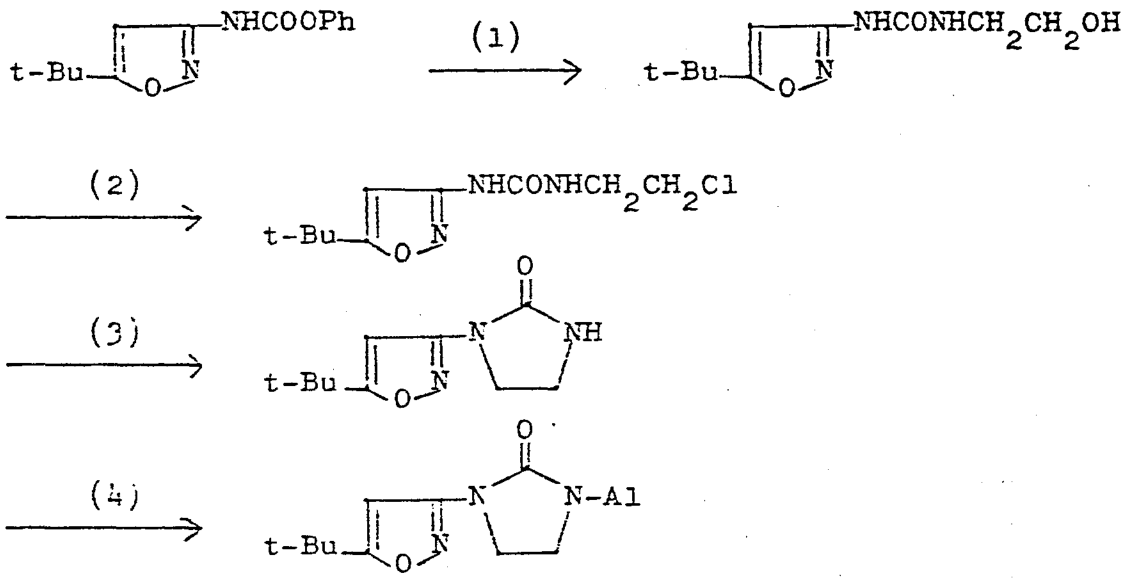


(1) To a solution of 2.60 g of phenyl 5-t-butylisoxazole-3-carbamate dissolved in 20 ml of methylene chloride is added 3.67 g of ethanolamine, and the mixture is stirred overnight at room temperature. The reaction mixture is concentrated in vacuo, and the residue is purified by chromatography on silica gel to give 4.46 g of the objective compound as crystals. Yield, 98.1 %.

This is recrystallized from benzene to give 1-(2-hydroxyethyl)-3-(5-t-butyl-3-isoxazolyl)urea as colorless plates, m.p. 108.5 - 109.5°C.

(2) To 22.73 g of the compound prepared in the above

step (1) are added 175 ml of dry benzene and 14.28 g of thionyl chloride and the mixture is refluxed under heating for half an hour. The reaction mixture is concentrated in vacuo, and the residue is purified by chromatography on silica gel to give 23.17 g of the objective compound as crystals. Yield, 94.3 %.

This is recrystallized from benzene to give 1-(2-chloroethyl)-3-(5-t-butyl-3-isoxazolyl)urea as colorless needles, m.p. 146.5 - 147.5°C.

(3) To 2.46 g of the compound prepared in the step (2) are added 11.22 g of 15 % aqueous potassium hydroxide and 6 ml of pyridine, and the mixture is stirred at room temperature for two hours. The reaction mixture is adjusted at pH 3 by adding hydrochloric acid and extracted with chloroform. The chloroform layer is washed with 2 % hydrochloric acid and water in order, dried over anhydrous sodium sulfate and concentrated in vacuo to give 2.02 g of the objective compound as crystals. Yield, 96.5 %.

This is recrystallized from hexane-benzene to give 1-(5-t-butyl-3-isoxazolyl)-2-imidazolidinone as colorless prisms. m.p.: 167 - 169°C.

(4) To a mixture of 1.26 g of the compound prepared in (3) and 22 ml of dry dimethylformamide is added 0.25 g of 63.7 % sodium hydride (suspension in mineral oil) and the mixture is stirred at room temperature for half

an hour. To the reaction mixture is added a mixture of 1.21 g of allyl iodide with 3 ml of dried dimethylformamide at a temperature of lower than 15°C, and then the reaction mixture is stirred for an hour and concentrated in vacuo. The residue is mixed with 50 ml of 1 % hydrochloric acid and then the mixture is extracted with methylene chloride. The organic layer is dried over anhydrous sodium sulfate, and then concentrated in vacuo. The residue is purified by chromatography on silica gel to give 1.52 g of the objective compound as crystals. This is recrystallized from hexane to give 1-allyl-3-(5-t-butyl-3-isoxazolyl)-2-imidazolidinone as colorless needles, m.p. 55 - 56°C.

Example 9

[Production of 3-(5-t-butyl-3-isoxazolyl)-1-methyl-2-imidazolidinone]

To a mixture of 0.4185 g of 1-(5-t-butyl-3-isoxazolyl)-2-imidazolidinone prepared in Example 8 (2) with 4 ml of dry dimethylformamide is added 0.088 g of 60 % aqueous sodium hydride, and then the mixture is stirred at room temperature for an hour. To the mixture is added a mixture of 0.34 g of 93 % methyl iodide with 3 ml of dry dimethylformamide, and then the mixture is stirred at room temperature for an hour. The reaction mixture is concentrated in vacuo, and the residue is mixed with 10 ml of water and extracted with methylene chloride. The organic layer is

dried over anhydrous sodium sulfate and then concentrated in vacuo. The residue is purified by chromatography on silica gel to give 0.4421 g of the objective compound as crystals. Yield, 99.0 %.

Example 10

[Production of 1-(5-t-butyl-3-isoxazolyl)-3-methyl-1,2,3,4,5,6-hexahydropyrimidin-2-one]

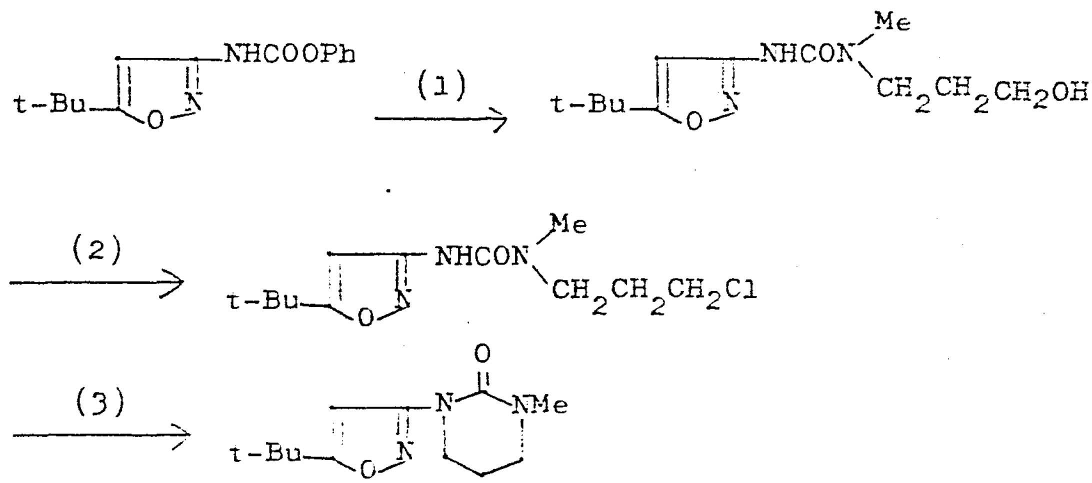


(1) To 2.60 g of phenyl 5-t-butyl-3-isoxazolylcarbamate is added a mixture of 15 ml of methylene chloride and 1.78 g of N-methyl-3-hydroxypropylamine, and the mixture is allowed to stand at room temperature for two days. The reaction mixture is concentrated in vacuo and the residue is purified by chromatography on silica gel to give 2.26 g of the objective compound as crystals. Yield, 88.5 %. This is recrystallized from cyclohexane-benzene to give 1-(3-hydroxypropyl)-1-methyl-3-(5-t-butyl-3-isoxazolyl)urea as colorless prisms, m.p. 106.0 - 107.0°C.

(2) To 1.28 g of the compound prepared in the above (1) are added 5 ml of dry benzene and 0.71 g of thionyl chloride and then the mixture is refluxed for half an hour. The reaction mixture is concentrated in vacuo and the residue is purified by chromatography on silica gel to give 1.17 g of the objective compound as crystals. Yield, 85.5 %. This is recrystallized from hexane to give 1-(3-chloropropyl)-1-methyl-3-(5-t-butyl-3-isoxazolyl)urea as pale-brown plates, m.p. 94.3 - 95.3°C.

(3) To 237.88 mg of the compound prepared in the above (2) are added 0.87 ml of pyridine and 980 mg of 15 % aqueous potassium hydroxide, and then the mixture is stirred at room temperature for an hour. The mixture is adjusted to pH 3 with 5 % hydrochloric acid and then extracted with methylene chloride. The organic layer is dried and then concentrated in vacuo. The residue is purified by chromatography on silica gel to give 184.72 mg of the objective compound as crystals. Yield, 89.6 %. This is recrystallized from hexane to give pale-yellowish crystals, m.p. 57.0 - 58.0°C.

Example 11

[Production of 1-(5-t-butyl-4-chloro-3-isoxazolyl)-3-methyl-1,2,3,4,5,6-hexahydropyrimidin-2-one]

a) In the same manner as in Example 10, the following compounds are prepared.

| | Amount of Starting material (mol) | Yield (g) (%) | m.p. (°C) Appearance |
|---|---|---|---|
| 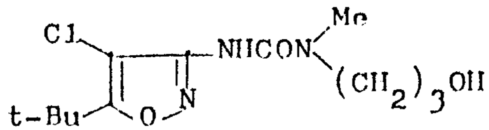 | 0.0140 | 3.86<br>95.2 | 115.5-116.5<br>colorless needles |
| 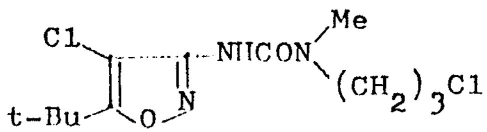 | 0.006 | 1.13<br>61.0 | —<br>colorless oil* |
| 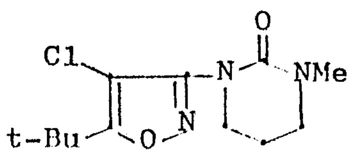 | 0.00357 | 0.96<br>98.7 | 82.0-85.0<br>colorless plates |

* IR ($CHCl_3$) $cm^{-1}$ : 1696, 1528, 1480

b) Alternative route:

To a mixture of 0.57 g of the product in Example 10 and 2.5 ml of methylene chloride is added a solution of chlorine gas (0.00280 mol) in 2.8 ml of carbon tetrachloride and the resulting mixture is stirred at room temperature for five minutes. The reaction mixture is concentrated in vacuo to give 0.72 g of the objective compound as crude crystals.

Example 12

[Production of 1-(5-t-butyl-3-isoxazolyl)-3-methyl-1,2,3,4,5,6-hexahydropyrimidin-2-one]

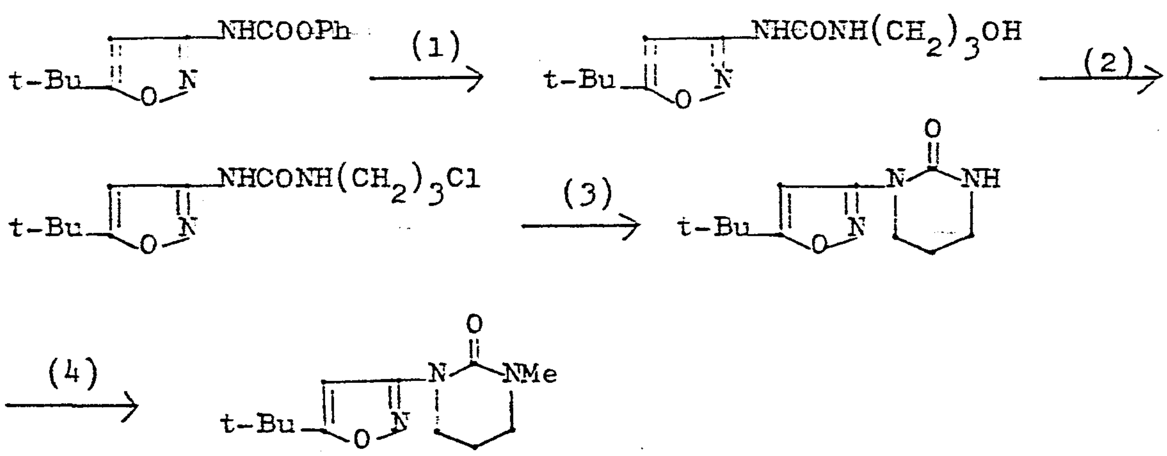


(1) To a solution of 2.60 g of phenyl 5-t-butyl-3-isoxazolylcarbamate in 15 ml of methylene chloride are added 0.90 g of 3-hydroxypropylamine and 1.01 g of triethylamine and the mixture is stirred overnight at room temperature. 2.5 % Hydrochloric acid (50 ml) is added and the mixture is extracted with methylene chloride. The organic layer is dried over anhydrous sodium sulfate and then condensed in vacuo. The residue is purified by chromatography on silica

gel to give 2.41 g of the product as crystals. Yield, 99.9 %. This is recrystallized from ethyl acetate-cyclohexane to give 1-(3-hydroxypropyl)-3-(5-t-butyl-3-isoxazolyl)-urea as colorless needles, m.p. 91 - 92°C.

(2) The product prepared in the above (1) is treated with thionyl chloride in dry benzene. The reaction is carried out in the same manner as in Example 10 (2), and then the crystals obtained as the objective product are recrystallized from cyclohexane-hexane to give 1-(3-chloropropyl)-3-(5-t-butyl-3-isoxazolyl)urea as colorless needles, m.p. 82.5 - 83.5°C. Yield, 96.0 %.

(3) To 1.30 g of the product prepared in the above (2) are added 15 ml of water, 1.40 g of potassium hydroxide and 3.0 ml of pyridine, and the mixture is stirred at room temperature for two hours. The reaction mixture is adjusted to pH 2 with dilute hydrochloric acid and extracted with methylene chloride. The methylene chloride layer is dried over anhydrous sodium sulfate and concentrated in vacuo. The residue is recrystallized from ethyl acetate-cyclohexane to give 0.93 g of 1-(5-t-butyl-3-isoxazolyl)-1,2,3,4,5,6-hexahydropyrimidin-2-one as colorless granular crystals, m.p. 164 - 165°C. Yield, 83.3 %.

(4) To a mixture of 2.23 g of the product prepared in the above (3) in 38 ml of dry dimethylformamide is added 0.41 g of 63.7 % sodium hydride and then the mixture is stirred at

room temperature for half an hour. A mixture of 93 % methyl iodide (1.27 g) and 3 ml of dimethylformamide is added thereto and the reaction mixture is stirred at room temperature for 3 hours and evaporated in vacuo. 1 % Hydrochloric acid (100 ml) is added to the residue and the mixture is extracted with methylene chloride. The organic layer is dried and then concentrated in vacuo. The residue is purified by chromatography on silica gel to give 1.59 g of the product as crystals. Recrystallization from hexane gives colorless needles, m.p. 58 - 59°C. Yield, 67.1 %.

Example 13

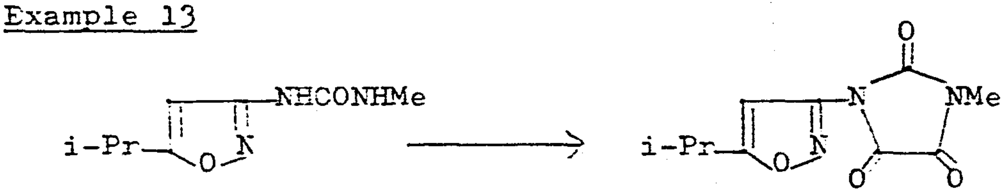

[Production of 1-methyl-3-(5-isopropyl-3-isoxazolyl)-2,4,5-trioxo-imidazolidine]

To 3.00 g of 1-methyl-3-(5-isopropyl-3-isoxazolyl)-urea are added 33 ml of dry benzene and 2.50 g of oxalyl chloride, and the mixture is refluxed for an hour. The reaction mixture is concentrated in vacuo and the residue is recrystallized from a mixture of benzene and cyclohexane to give 3.58 g (Yield, 92.0 %) of colorless crystals, m.p. 140.5 - 141.0°C.

Example 14 - 24

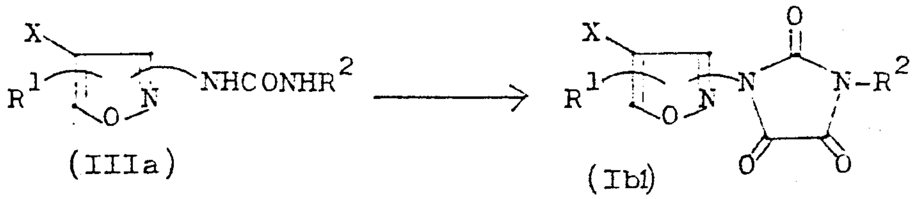

(IIIa) (Ib1)

The objective products (Ib1) are prepared from the starting material (IIIa) in the same manner as in Example 13. The results are shown in Table 2.

Table 2

| Example Nos. | $R^1$ | $R^2$ | X | Amount of Starting Material (IIIa) (g) | Yield (g) | Yield (%) | m.p. (°C) | Appearance |
|---|---|---|---|---|---|---|---|---|
| 14 | 5-t-Bu | Me | H | 7.89 | 9.92 | 98.7 | 127.5 - 128.5 | colorless prisms |
| 15 | " | " | Cl | 2.55 | 2.95 | 93.9 | 99.0 - 100.5 | colorless needles |
| 16 | " | " | Br | 0.83 | 0.89 | 89.9 | 137.5 - 138.5 | colorless prisms |
| 17 | 3-t-Bu | " | H | 2.76 | 3.24 | 92.1 | 164.0 - 166.0 | colorless needles |
| 18 | " | " | Cl | 1.04 | 1.21 | 94.1 | 133.0 - 134.0 | " |
| 19 | " | " | Br | 0.83 | 0.92 | 92.9 | 132.5 - 133.5 | colorless plates |
| 20 | 3-i-Pr | " | H | 3.31 | 3.78 | 88.0 | 127.0 - 127.5 | colorless powdery crystals |
| 21 | 5-t-Bu | Al | " | 1.00 | 1.23 | 98.6 | 110.0 - 111.0 | colorless plates |
| 22 | " | H | " | 2.51 | 2.99 | 92.0 | 150.5 - 152.0 | colorless needles |
| 23 | 3-t-Bu | " | " | 4.03 | 3.56 | 68.2 | 161.0 - 162.0 | pale-yellow prisms |
| 24 | 3-i-Pr | " | " | 5.75 | 4.36 | 57.5 | 141.0 - 142.0 | pale brown prisms |

Example 25

[Production of 1-methyl-3-(5-t-butyl-3-isoxazolyl)-2,4,5-trioxo-imidazolidine] (Alternative route)

In the same manner as in Example 9 is treated 0.4744 g of the product in Example 22. The reaction product is washed with hexane without purification by chromatography and then dissolved in benzene to remove insoluble materials. Removal of benzene by evaporation gives 0.4783 g (Yield, 95.2 %) of the objective product as crystals.

Example 26

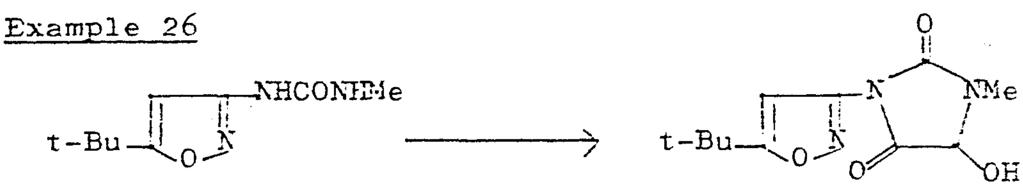

[Production of 1-methyl-3-(5-t-butyl-3-isoxazolyl)-5-hydroxy-2,4-dioxo-imidazolidine]

To 17.25 g of 1-methyl-3-(5-t-butyl-3-isoxazolyl)urea are added 8.86 g of glyoxylic acid monohydrate and 105 ml of chloroform and the mixture is subjected to azeotropic distillation for three hours in order to remove the water produced. The reaction mixture is concentrated in vacuo and the residue is purified by chromatography on silica gel to give 16.62 g (Yield, 75.0 %) of the product. This is recrystallized from a mixture of cyclohexane and benzene, and then dried in vacuo at 80°C for eight hours to eliminate the crystal benzene, whereby colorless

powdery crystals, m.p. 118.0 - 120.5°C, are obtained (The structural formula was confirmed by X-ray analysis on crystals of the acetate.). Additionally 4.90 g (yield, 22.1 %) of 1-methyl-3-(5-t-butyl-3-isoxazolyl)-4-hydroxy-2,5-dioxo-imidazolidine is produced as a by-product.

Examples 27 - 29

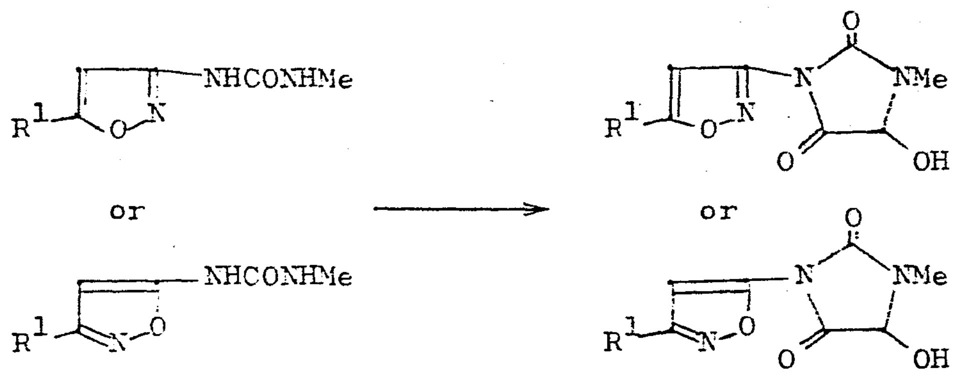

or

or

(IIIa)

(Ib)

The starting material (IIIa) is treated in the same manner as in Example 26 to give the objective product (Ib). The results are summarized in Table 3.

Table 3

| Example Nos. | $R^1$ | Amount of IIIa (g) | Yield (g) | Yield (%) | m.p. (°C)/IR($CHCl_3$) $cm^{-1}$ | Appearance |
|---|---|---|---|---|---|---|
| 27 | 5-i-Pr | 5.04 | 5.06 | 76.9 | 137.0-138.0 | colorless scales |
| 28 | 3-t-Bu | 4.93 | 3.06 | 48.3 | 112.0-113.0 | colorless prisms |
| 29 | 3-i-Pr | 5.04 | 3.44 | 48.2 | 1750, 1465, 1400 | yellow-green oil |

Example 30

[Production of 1-methyl-3-(5-t-butyl-4-chloro-3-isoxazolyl)-4-hydroxy-2,5-dioxo-imidazolidine]

In the same manner as in Example 26 is treated 1.85 g of 1-methyl-3-(5-t-butyl-4-chloro-3-isoxazolyl)urea to give 0.864 g (yield, 37.5 %) of the product as a colorless oil.

IR ($CHCl_3$) 1738, 1460, 1100 $cm^{-1}$

Example 31

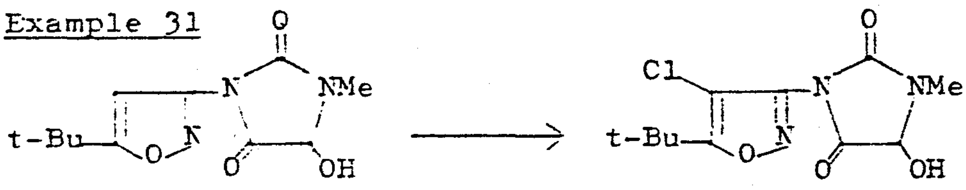

[Production of 1-methyl-3-(5-t-butyl-4-chloro-isoxazolyl)-5-hydroxy-2,4-dioxo-imidazolidine]

To 1.52 g of 1-methyl-3-(5-t-butyl-3-isoxazolyl)-5-hydroxy-2,4-dioxo-imidazolidine are added 6 ml of methylene chloride and 1.04 g of pyridine. A solution of chlorine gas (1.81 mol/l) in carbon tetrachloride (3.65 ml) is then added under ice-cooling, and is stirred at room temperature for half an hour. This procedure is repeated again. After addition of chloroform, the reaction mixture is washed with 5 % hydrochloric acid and water in order, dried over anhydrous sodium sulfate and then concentrated in vacuo. The residue is purified by chromatography on silica gel to give 0.90 g (yield, 51.9 %) of the product as pale-yellow oil.

IR ($CHCl_3$) 1750, 1449, 1408 $cm^{-1}$

Example 32

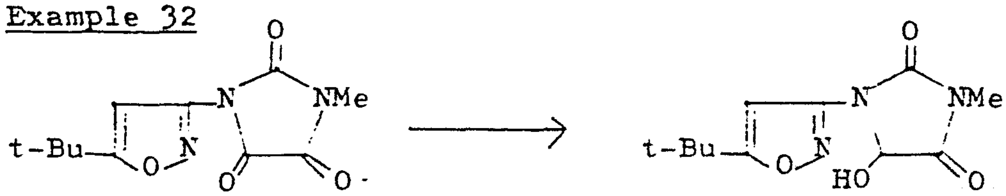

[Production of 1-methyl-3-(5-t-butyl-3-isoxazolyl)-4-hydroxy-2,5-dioxo-imidazolidine]

To 1.26 g of the product in Example 14 is added 5 ml of methanol, the mixture is cooled to -5°C, and then 48.4 mg of sodium borohydride is gradually added. The mixture is subsequently stirred at room temperature for 30 minutes and then concentrated in vacuo. 1 % Hydrochloric acid is added thereto, and the mixture is extracted with methylene chloride. The organic layer is dried over anhydrous sodium sulfate, and concentrated in vacuo. The residue is purified by chromatography on silica gel to give 1.06 g (yield, 83.4 %) of the product as crystals. The product is recrystallized from benzene and dried under heating in vacuo at 90°C for 30 minutes to remove crystalline benzene, whereby colorless powdery crystals, m.p. 142.5 - 143.5°C, are obtained.

Examples 33 - 36

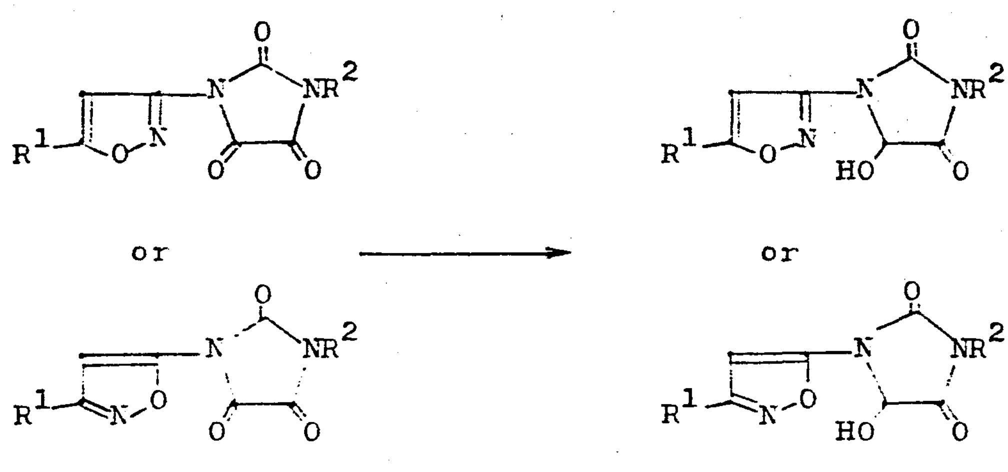

or

or

(Ib1) (Ic)

The starting material (Ib1) is treated in the same manner as in Example 32 to give the objective product (Ic). The results are summarized in Table 4.

Table 4

| Example Nos. | $R^1$ | $R^2$ | Amount of Ibi used (g) | Yield (g) | Yield (%) | m.p. (°C) | Appearance |
|---|---|---|---|---|---|---|---|
| 33 | 5-i-Pr | Me | 10.67 | 4.47 | 41.0 | 121.0-122.0 | colorless prisms |
| 34 | 3-t-Bu | " | 11.31 | 5.56 | 48.8 | 151.0-152.0 | colorless powdery crystals |
| 35 | 3-i-Pr | " | 10.67 | 7.88 | 73.2 | 136.0-137.0 | " |
| 36 | 5-t-Bu | H | 2.53 | 1.71 | 67.0 | 173.0-174.5(dec.) | colorless prisms |

Example 36′

[Production of 3-(3-t-butyl-5-isoxazolyl)-4-hydroxy-2,5-dioxo-imidazolidine]

In the same manner as in Example 32 except using tetrahydrofuran (20 ml) in place of methanol is treated 1.19 g of the product obtained in Example 23 to give 0.66 g (yield, 55.2 %) of the objective product as crystals. The product is recrystallized from methanol-benzene to give colorless plates, m.p. 227 - 228°C.

Example 37

[Production of 1-methyl-3-(5-t-butyl-3-isoxazolyl)-4-hydroxy-2,5-dioxo-imidazolidine] (Alternative route···1)

To 253.3 mg of the product prepared in Example 26 is added 4 ml of dry tetrahydrofuran, and then 115.7 mg of 97 % potassium t-butoxide is added to the mixture under ice-cooling. After stirring for 30 minutes at room temperature, the reaction mixture is poured into 1 % hydrochloric acid which is ice-cooled in advance. The resulting solution is concentrated in vacuo to remove the tetrahydrofuran. The residual aqueous solution is extracted with benzene, and the organic layer is dried over anhydrous sodium sulfate and then concentrated in vacuo. The residue is purified by chromatography on silica gel to give 81.9 mg (yield,32.3 %) of the product as crystals.

Example 38

[Production of 1-methyl-3-(5-t-butyl-3-isoxazolyl)-4-hydroxy-2,5-dioxo-imidazolidine] (Alternative route···2)

In the same manner as in Example 9, 119.6 mg of the product in Example 36 is treated to give 86.9 mg (yield, 68.6 %) of the objective product as crystals.

Example 39

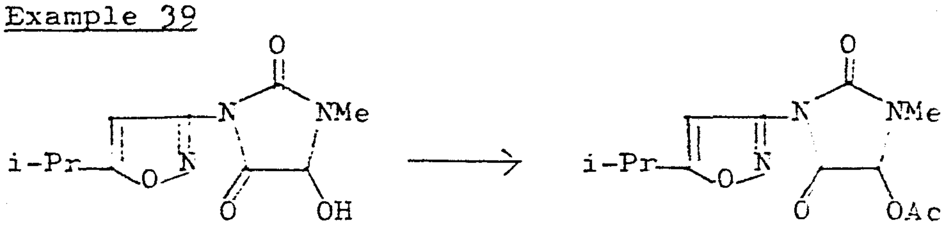

[Production of 1-methyl-3-(5-isopropyl-3-isoxazolyl)-5-acetoxy-2,4-dioxo-imidazolidine]

To a mixture of 1.00 g of the product in Example 27, 8.4 ml of dry benzene and 0.46 g of pyridine is added 0.43 g of acetyl chloride under ice-cooling, and the mixture is stirred under ice-cooling for 30 minutes and then at room temperature for additional 30 minutes. Water is added into the reaction mixture and the mixture is extracted with ethyl acetate. The ethyl acetate layer is washed with 5 % hydrochloric acid, 8 % aqueous sodium hydrogen--carbonate and water in order and concentrated in vacuo to give 1.12 g (yield,95.5 %) of the objective compound as a yellowish-green oil.

IR ($CHCl_3$) 1753, 1449, 1401 $cm^{-1}$.

Example 40 - 58

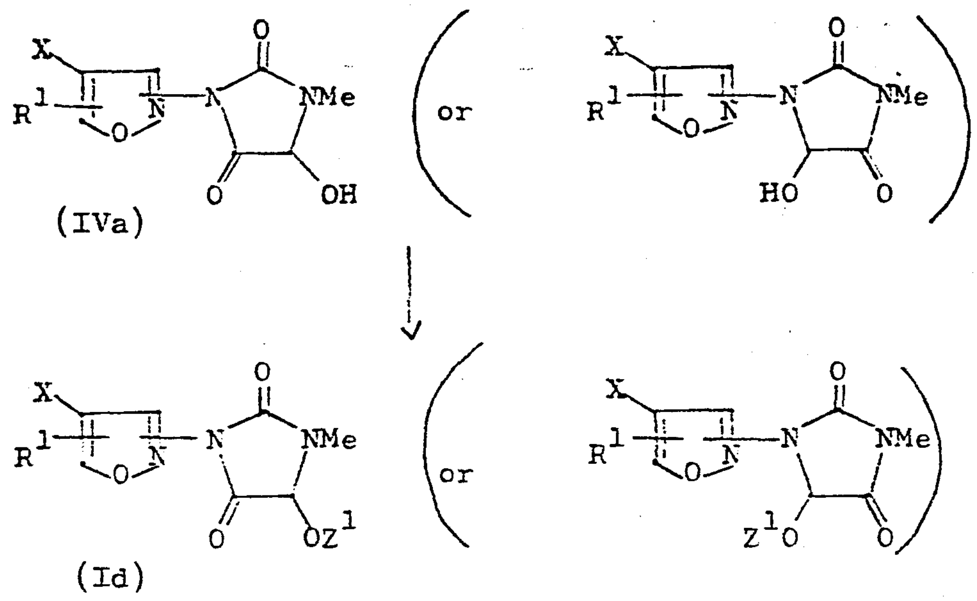

(IVa) or

(Id) or

The starting material (IVa) is treated in the same manner as in Example 39 to give the product (Id). The results are summarized in Table 5.

Table 5

| Example Nos. | $R^1$ | $Z^1$ | X | Amount of IVa (g) | Yield(g) | Yield(%) | m.p.(°C)/IR($CHCl_3$)$cm^{-1}$ | Appearance |
|---|---|---|---|---|---|---|---|---|
| 40 | 5-t-Bu | MeCO | H | 5-OH 0.76 | 0.52 | 58.8 | 124-126 | yellow fine prisms |
| 41 | " | " | Cl | " 1.15 | 1.10 | 83.3 | 1758, 1440, 1399 | pale-brown oil |
| 42 | 3-t-Bu | " | H | " 1.79 | 0.794 | 41.8 | 1762, 1404, 1376 | colorless oil |
| 43 | 3-i-Pr | " | " | " 1.30 | 0.542 | 38.5 | 1760, 1405, 1378 | yellowish-green oil |
| 44 | 5-t-Bu | n-PrCO | " | " 0.76 | 0.761 | 78.5 | 1753, 1449, 1400 | colorless oil |
| 45 | " | " | Cl | " 1.15 | 1.38 | 96.4 | 1752, 1440, 1399 | pale-brown oil |
| 46 | 5-i-Pr | " | H | " 1.00 | 0.855 | 66.1 | 1760, 1457, 1409 | colorless oil |
| 47 | 3-t-Bu | " | " | " 1.01 | 1.31 | 101.3* | 1760, 1467, 1405 | pale-brown oil |
| 48 | 3-i-Pr | " | " | " 1.00 | 0.91 | 70.4 | 1760, 1468, 1403 | pale yellowish-brown oil |
| 49 | 5-t-Bu | PhCO | " | " 0.76 | 0.81 | 78.6 | 1745, 1440, 1055 | colorless oil |
| 50 | " | 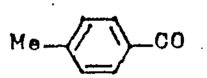 | " | " 0.76 | 0.808 | 72.5 | 1754, 1450, 1076 | " |
| 51 | " | 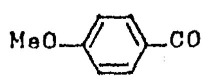 | " | " 0.76 | 0.774 | 68.2 | 1754, 1606, 1078 | " |
| 52 | " | PhCO | Cl | " 1.15 | 0.95 | 60.6 | 130.0-132.0 | colorless prisms |
| 53 | 5-i-Pr | " | H | " 1.00 | 0.954 | 66.5 | 105.0-106.5 | colorless pillars |
| 54 | 3-t-Bu | " | " | " 0.843 | 0.791 | 76.9 | 143.0-144.0 | colorless needles |
| 55 | 3-i-Pr | " | " | " 3.04 | 0.391 | 8.5 | 83.5-89.0 | pale yellow crystals |
| 56 | 5-i-Pr | MeCO | " | 4-OH 1.00 | 0.715 | 60.8 | 143.3-144.5 | colorless needles |
| 57 | " | n-PrCO | " | " 1.00 | 1.186 | 91.7 | 1740, 1602, 1452 | colorless oil |
| 58 | 5-t-Bu | " | " | " 0.76 | 0.941 | 97.1 | 104.0-105.0 | colorless needles |

* Crude yield

Example 59

[Production of 1-methyl-3-(5-t-butyl-3-isoxazolyl)-5-acetoxy-2,4-dioxo-imidazolidine] (Alternative route)

To 1.55 g of the product in Example 26 are added 12 ml of dry benzene, 1.36 g of triethylamine and 1.25 g of acetic anhydride and the mixture is refluxed for one hour and 20 minutes. The reaction mixture is mixed with water, stirred at room temperature for an hour and extracted with ethyl acetate. The ethyl acetate layer is washed with 5 % hydrochloric acid, 8 % sodium hydrogencarbonate and water in order, and then concentrated in vacuo. The residue is purified by chromatography on silica gel to give 0.42 g (yield, 25.6 %) of the product as crystals.

Example 60

[Production of 1-methyl-3-(5-t-butyl-3-isoxazolyl)-4-acetoxy-2,5-dioxo-imidazolidine]

In the same manner as in Example 59 is treated 1.31 g of the product in Example 32 to give 0.54 g (yield, 32.8 %) of the objective compound as crystals. The crystals are recrystallized from a mixture of benzene and cyclohexane to give colorless needles, m.p. 154.7 - 156.0°C.

Example 61

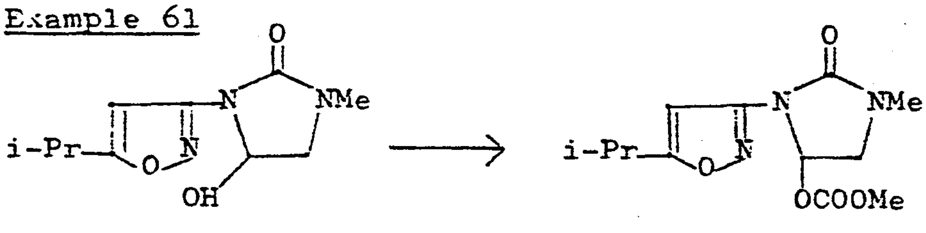

[Production of 1-methyl-3-(5-isopropyl-3-isoxazolyl)-4-methoxycarbonyloxy-2-imidazolidinone]

To a mixture of 2.25 g of 1-methyl-3-(5-isopropyl-3-isoxazolyl)-4-hydroxy-2-imidazolidinone and 40 ml of dry tetrahydrofuran cooled to -78°C is added 1.16 g of 97 % potassium t-butoxide and the mixture is stirred for 30 minutes. Then 0.945 g of methyl chloroformate is added and the mixture is stirred for 30 minutes, then warmed up to room temperature, and concentrated in vacuo. The residue is combined with water and then extracted with benzene. The benzene layer is dried over anhydrous sodium sulfate and concentrated in vacuo. The residue is purified by chromatography on silica gel to give 1.37 g (yield, 48.4 %) of the objective compound as a colorless oil.

IR ($CHCl_3$) 1742, 1284, 1446 $cm^{-1}$

Examples 62 - 71

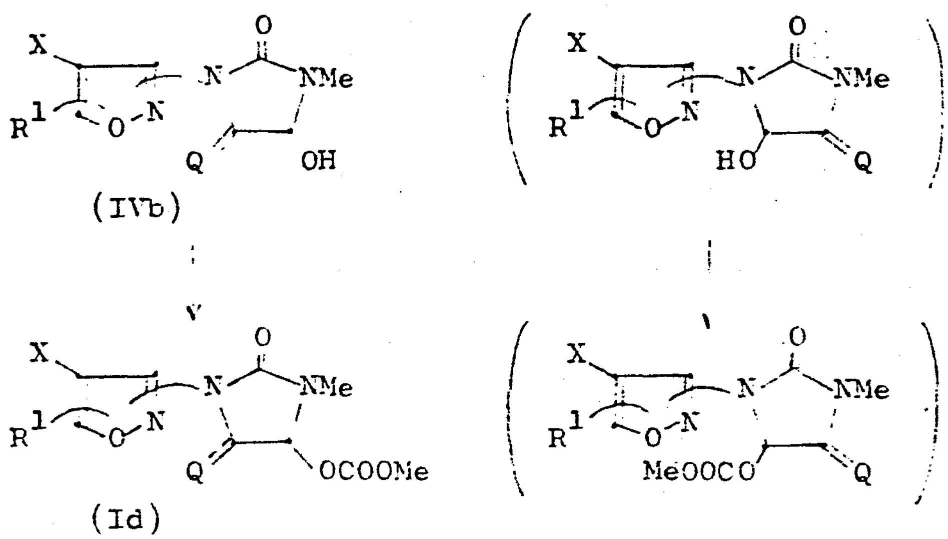

(IVb)

(Id)

The starting material (IVb) is treated in the same manner as in Example 61 to give the product (Id). The results are summarized in Table 6.

Table 6

| Example Nos. | $R^1$ | X | IVb | | | Yield (g) | Yield (%) | m.p.(°C)/IR($CHCl_3$)$cm^{-1}$ | Appearance |
|---|---|---|---|---|---|---|---|---|---|
| | | | Q | Position of -OH | Amount of IVb (g) | | | | |
| 62 | 5-t-Bu | H | $H_2$ | 4 | 0.957 | 1.109 | 93.2 | 1735, 1437, 1275 | pale-yellow oil |
| 63 | " | Cl | " | " | 1.37 | 1.24 | 74.9 | 1740, 1440, 1278 | colorless oil |
| 64 | 3-t-Bu | H | " | " | 1.91 | 1.82 | 76.5 | 1742, 1446, 1284 | " |
| 65 | 3-i-Pr | " | " | " | 1.80 | 1.66 | 73.5 | 1743, 1611, 1275 | " |
| 66 | 5-t-Bu | " | O | 5 | 1.27 | 1.10 | 70.7 | 118.5-120.0 | colorless prisms |
| 67 | " | Cl | " | " | 1.73 | 1.59 | 76.6 | 161.5-163.5 | " |
| 68 | 5-i-Pr | H | " | " | 1.44 | 1.32 | 73.8 | 1760, 1447, 1260 | pale-yellow oil |
| 69 | 3-t-Bu | " | " | " | 1.52 | 1.19 | 63.7 | 1760, 1405, 1260 | colorless oil |
| 70 | " | " | " | 4 | 1.52 | 1.67 | 89.5 | 146.5-147.5 | colorless needles |
| 71 | 5-t-Bu | " | " | " | 1.27 | 1.02 | 65.3 | 141.0-142.5 | " |

Example 72

[Production of 1-methyl-3-(5-t-butyl-3-isoxazolyl)-4-methoxycarbonyloxy-2,5-dioxo-imidazolidine] (Alternative route)

To a mixture of 1.27 g of the product in Example 26 and 20 ml of dry tetrahydrofuran is added 0.5785 g of 97 % potassium t-butoxide under ice-cooling, and then the resultant mixture is stirred at room temperature for 30 minutes. Then 0.4725 g of methyl chloroformate is added under ice-cooling, and the mixture is stirred at room temperature for 30 minutes and then concentrated in vacuo. The residue is mixed with water and extracted with methylene chloride. The organic layer is dried over anhydrous sodium sulfate and concentrated in vacuo. The resultant residue is purified by chromatography on silica gel to give 0.75 g (yield, 47.9 %) of the objective product as crystals.

Example 73

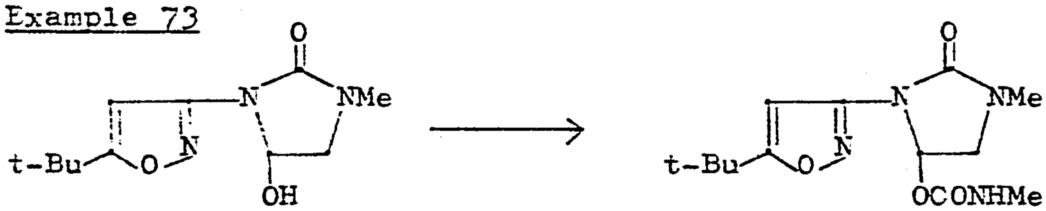

[Production of 1-methyl-3-(5-t-butyl-3-isoxazolyl)-4-(N-methylcarbamoyloxy)-2-imidazolidinone]

To 1.00 g of 1-methyl-3-(5-t-butyl-3-isoxazolyl)-4-hydroxy-2-imidazolidinone are added 0.72 g of methyl isocyanate and three drops of thriethylamine, and the mixture is allowed to stand at room temperature for 16 hours.

Then methyl isocyanate remaining unchanged is removed by distillation in vacuo. The residue is purified by chromatography on silica gel to give 1.17 g (yield, 88.3 %) of the objective product as colorless vitriform solid, m.p. 129.5 - 133.0°C.

Example 74 - 78

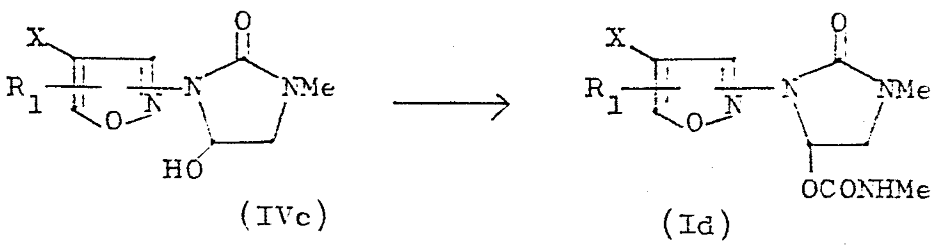

(IVc) (Id)

The starting material (IVc) is treated in the same manner as in Example 73 to give the objective product (Id). The results are summarized in Table 7.

Table 7

| Example Nos. | $R^1$ | X | Amount of starting material IVc (g) | Yield (g) | Yield (%) | m.p. (°C)/IR($CHCl_3$)$cm^{-1}$ | Appearance |
|---|---|---|---|---|---|---|---|
| 75 | 5-t-Bu | Cl | 1.37 | 1.72 | 104.3* | 1726, 1434, 1116 | colorless oil |
| 76 | 5-i-Pr | H | 1.13 | 0.97 | 68.7 | 132.0-133.5 | colorless pillars |
| 77 | 3-t-Bu | H | 1.20 | 1.44 | 97.2 | 106.0-108.5 | colorless prisms |
| 78 | 3-i-Pr | H | 1.13 | 1.40 | 99.2 | 115.5-116.5 | " |

* Crude yield

Example 79

[Production of 1-methyl-3-(5-t-butyl-3-isoxazolyl)-5-(N-methylcarbamoyloxy)-2,4-dioxo-imidazolidine]

To 0.84 g of the product in Example 26 is added 0.56 g of methyl isocyanate and the mixture is heated in a sealed tube at 60°C for a week, and then the unreacted methyl isocyanate is distilled off in vacuo. The residue is recrystallized from 1,2-dichloroethane, then dried in vacuo at 95°C for 2 hours to remove the solvent of crystallization, whereby 0.63 g (yield, 61.8 %) of the objective product is obtained as colorless powdery crystals, m.p. 128.0 - 129.5°C.

Example 80 - 83

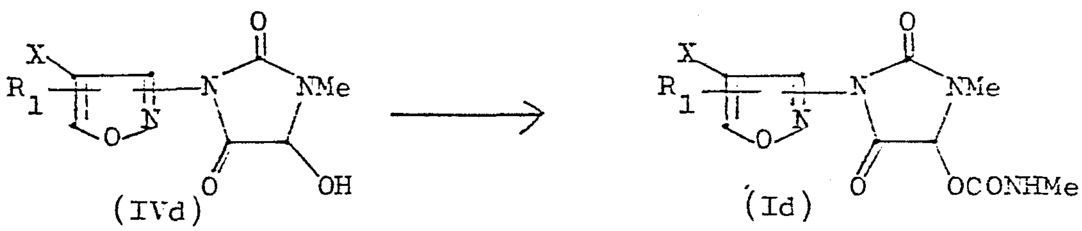

(IVd) (Id)

The starting material (IVd) is treated in the same manner as in Example 79 to give the objective product (Id). The results are summarized in Table 8.

Table 8

| Example Nos. | $R^1$ | X | Amount of starting material IVd (g) | Yield (g) | Yield (%) | m.p. (°C) | Appearance |
|---|---|---|---|---|---|---|---|
| 80 | 5-t-Bu | Cl | 4.03 | 2.54 | 52.6 | 177.5-178.0 | colorless plates |
| 81 | 5-i-Pr | H | 1.00 | 0.599 | 48.4 | 148.0-150.0 | colorless prisms |
| 82 | 3-t-Bu | H | 1.01 | 0.650 | 52.4 | 121.0-149.0 | colorless plates |
| 83 | 3-i-Pr | H | 1.00 | 0.820 | 66.2 | 139.5-140.5 | pale-yellow scales |

Example 84

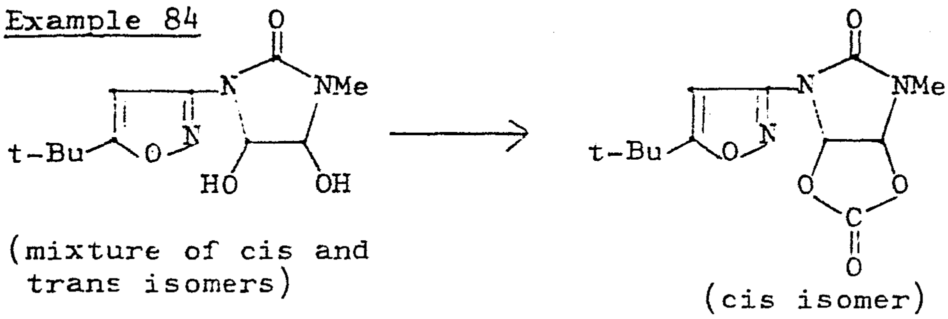

(mixture of cis and trans isomers)

(cis isomer)

[Production of cis-1-methyl-3-(5-t-butyl-3-isoxazolyl)-2-oxo-4,5-carbonyldioxy-imidazolidine]

To a mixture of 2.33 g of 1-methyl-3-(5-t-butyl-3-isoxazolyl)-4,5-dihydroxy-2-imidazolidinone (containing 65 % cis isomer) and 37 ml of dry chloroform is gradually added 2.96 g of 1,1'-carbonyldiimidazole over 5 hours. After termination of the addition the reaction mixture is stirred at room temperature for 30 minutes, then washed with water, dried over anhydrous sodium sulfate and then concentrated in vacuo. The residue is purified by chromatography on silica gel to give 1.48 g (yield, 57.6 %) of the objective compound as crystals. This is recrystallized from benzene to give colorless plates, m.p. 177.0 - 178.0°C (dec.).

Example 85

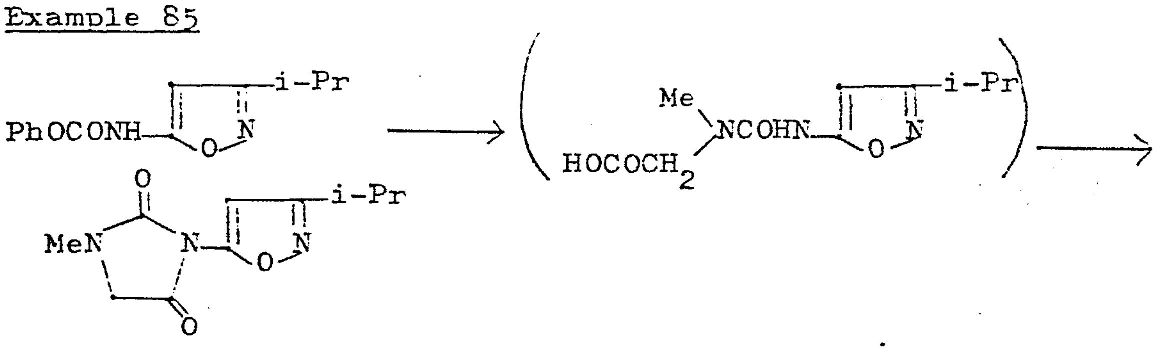

[Production of 1-methyl-3-(3-isopropyl-5-isoxazolyl)-2,4-dioxoimidazolidine]

To a solution of 12.31 g of phenyl 3-isopropylisoxazole-5-carbamate in 150 ml of methylene chloride are added 13.36 g of sarcosine and 15.18 g of triethylamine, and the mixture is stirred at room temperature for 2 hours. Then, 36 % hydrochloric acid (15.19 g) and 5 % hydrochloric acid (72.92 g) are added and the mixture is stirred at room temperature for 42 hours. The aqueous layer is separated and extracted with methylene chloride. The combined methylene chloride layers are dried over anhydrous sodium sulfate and concentrated in vacuo. The residue is purified by chromatography on silica gel to give 8.97 g (yield, 80.4 %) of the product as a pale yellow oil. IR ($CHCl_3$) 1800, 1740, 1408 $cm^{-1}$

Example 86 - 87

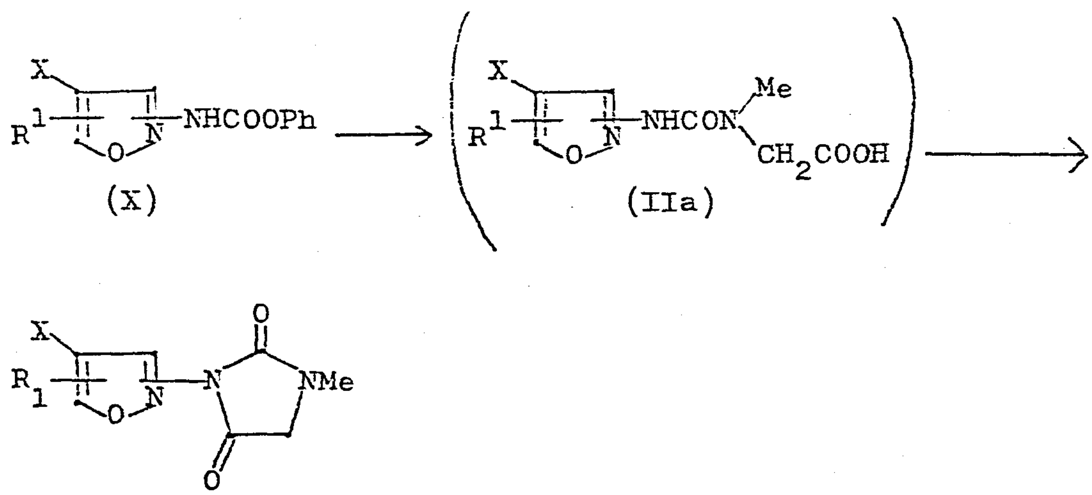

(X)

(IIa)

(Ia)

The starting material (X) is treated in the same manner as in Example 85 to give the objective product (Ia). The results are summarized in Table 9.

Table 9

| Example Nos. | $R^1$ | X | Amount of starting material (X) (g) | Yield (g) | Yield (%) | m.p. (°C) | Appearance |
|---|---|---|---|---|---|---|---|
| 86 | 5-t-Bu | Cl | 5.89 | 3.31 | 60.9 | 102.0 - 105.0 | colorless plates |
| 87 | 3-t-Bu | H | 6.76 | 1.17 | 19.0 | 98.5 - 99.0 | colorless needles |

Example 88

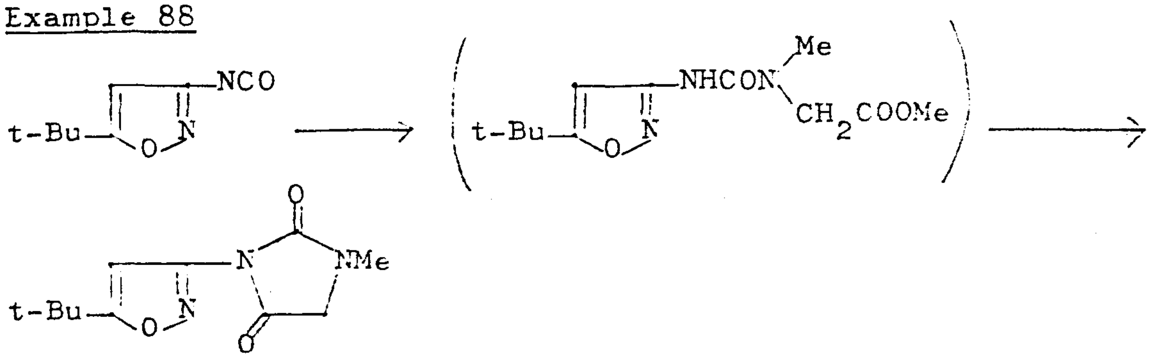

[Production of 1-methyl-3-(5-t-butyl-3-isoxazolyl)-2,4-dioxoimidazolidine]

To 9.97 g of 5-t-butyl-3-isoxazolyl isocyanate are added 120 ml of dry benzene and 6.11 g of methyl sarcosinate and the mixture is refluxed for 3 hours. The reaction mixture is concentrated in vacuo and the residue is purified by chromatography on silica gel to give 8.41 g (yield, 59.1 %) of the product as crystals. This is recrystallized from a mixture of benzene and cyclohexane to give colorless scales, m.p. 114.0 - 115.0°C.

Example 89

[Production of 1-methyl-3-(5-isopropyl-3-isoxazolyl)-2,4-dioxoimidazolidine]

In the same manner as in Example 88 is treated 18.26 g of 5-isopropyl-3-isoxazolyl isocyanate to give 19.74 g (yield, 73.7 %) of the objective product as crystals. This is recrystallized from a mixture of benzene and cyclohexane to give colorless needles, m.p. 97.0 - 98.0°C.

Example 90

[Production of 1-methyl-3-(5-t-butyl-4-chloro-3-isoxazolyl)-2.4-dioxoimidazolidine] (Alternative route)

To 0.95 g of the product prepared in Example 88 are added 4 ml of chloroform and chlorine gas (1 mole/l) in carbon tetrachloride (8.4 ml), then the mixture is allowed to stand at room temperature for five days, and concentrated in vacuo. The residue is purified by chromatography on silica gel to give 0.76 g (yield 70.3 %) of the product as crystals.

Example 91

[Production of 1-methyl-3-(5-isopropyl-4-chloro-3-isoxazolyl)-2,4-dioxoimidazolidine]

In the same manner as in Example 90 is treated 0.89 g of the product prepared in Example 89, whereby 0.86 g (yield. 83.9 %) of the objective product is obtained as a colorless oil.

IR ($CHCl_3$) 1800, 1740, 1448 $cm^{-1}$

Example 92

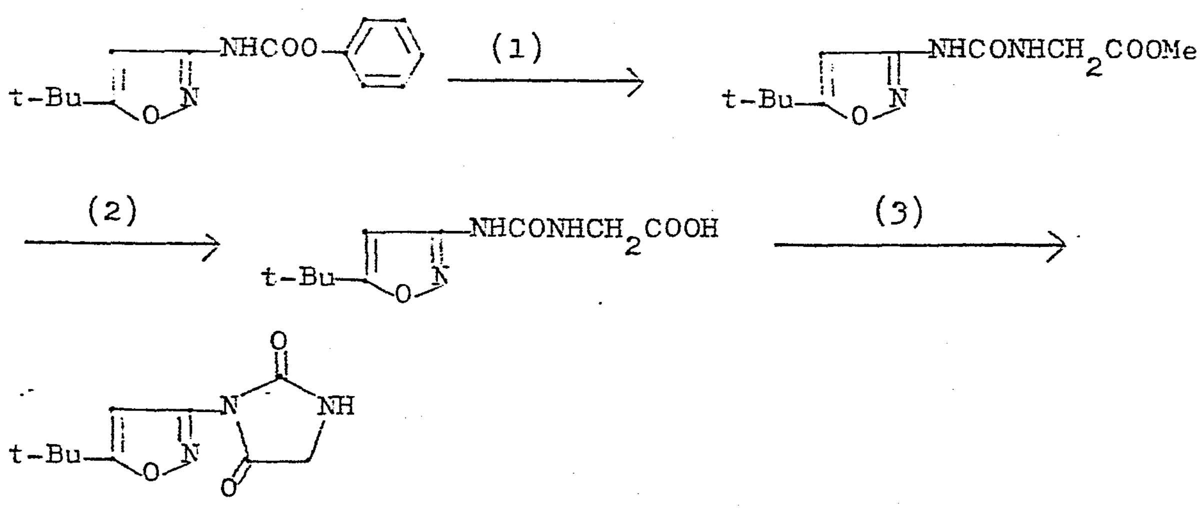

[Production of 1-(5-t-butyl-3-isoxazolyl)-2,5-dioxoimidazolidine]

(1) To a solution of 1.30 g of phenyl 5-t-butylisoxazole-3-carbamate in 20 ml of methylene chloride are added 1.26 g of methyl glycinate hydrochloride and 1.4 ml of triethylamine, and the mixture is stirred at room temperature for four days. Then water is added and the mixture is extracted with methylene chloride. The methylene chloride layer is dried over anhydrous sodium sulfate and concentrated in vacuo. The residue is purified by chromatography on silica gel to give 1.29 g of the product as crude crystals. This is recrystallized from a mixture of benzene-cyclohexane to give 1-methoxycarbonylmethyl-3-(5-t-butyl-3-isoxazolyl)urea as scales, m.p. 111 - 113°C.

(2) To 5.11 g of the product prepared in the above (1) is added 5 % aqueous sodium hydroxide (17.60 g), then the mixture is stirred at room temperature for an hour and adjusted to pH 1 with 36 % hydrochloric acid under ice-cooling. The precipitated crystals are collected by filtration and dried in vacuo at 80°C for eight hours to give 4.82 g (yield, 99.9 %) of the objective product as crystals. This is recrystallized from methanol to give 1-carboxymethyl-3-(5-t-butyl-3-isoxazolyl)urea as colorless plates, m.p. 201.0 - 203.0°C (dec.).

(3) To 1.45 g of the product prepared in the above (2)

are added 3 ml of dry benzene, 0.86 g of thionyl chloride and 0.06 ml of pyridine, the mixture is refluxed for 3 hours. After addition of additional 0.95 g of pyridine, the resultant mixture is further refluxed for 2 hours. The reaction mixture is concentrated in vacuo. Twenty ml of 5 % hydrochloric acid is added to the residue, and the mixture is extracted with methylene chloride. The methylene chloride layer is concentrated in vacuo, and the residue is purified by chromatography on silica gel to give 0.96 g (yield, 71.7 %) of the objective product as crystals. This is recrystallized from benzene to give 1-(5-t-butyl-3-isoxazolyl)-2,5-dioxoimidazolidine as colorless needles, m.p. 175.5 - 176.5°C.

Example 93 - 95

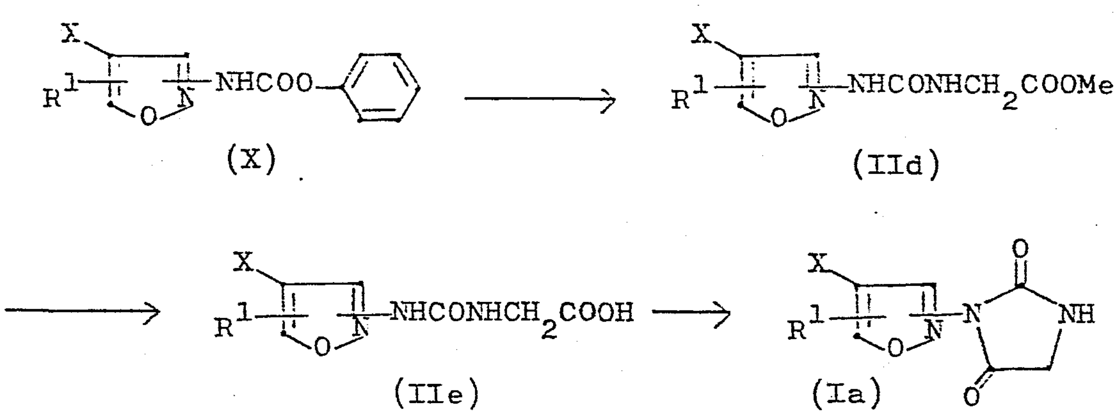

(X) (IId) (IIe) (Ia)

The starting material (X) is treated in the same manner as in Example 92 to give the objective product (Ia) as well as the intermediates (IId) and (IIe). The results are summarized in Table 10.

Table 10

| Example Nos. | $R^1$ | X | II d | II e | I a |
|---|---|---|---|---|---|
| | | | m.p.(°C)/IR($CHCl_3$)$cm^{-1}$ Appearance | m.p. (°C) Appearance | m.p. (°C) Appearance |
| 93 | 5-i-Pr | H | 94-95<br>colorless plates | 183.0-184.0(dec.)<br>colorless needles | 190.0-191.0<br>pale-brown needles |
| 94 | 3-t-Bu | H | 116.5-117.5<br>colorless needles | 175.0-176.0(dec.)<br>colorless prisms | 160.0-161.0<br>light-orange color needles |
| 95 | 3-i-Pr | H | 1745, 1708, 1550<br>pale-yellow oil | 132.5-133.5(dec.)<br>pale-yellow prisms | 115.0-116.0<br>pale-yellow needles |

Example 96

[Production of 1-(3-t-butyl-5-isoxazolyl)-2,5-dioxo-imidazolidine] (Alternative route)

To 0.12g of 1-carboxymethyl-3-(3-t-butyl-5-isoxazolyl)-urea are added 5 ml of dry tetrahydrofuran and 0.12 g of dicyclohexylcarbodiimide and the mixture is stirred at room temperature for 3 days. The precipitated crystals are filtrated off and the filtrate is concentrated in vacuo. The residue is purified by chromatography on silica gel to give 0.11 g of the objective product as crystals.

Example 97

[Production of 1-methyl-3-(5-t-butyl-3-isoxazolyl)-2,4-dioxoimidazolidine] (Alternative route)

In the same manner as in Example 9 except that the reaction temperature is altered to 0 - 5°C, is treated 0.4465 g of the product prepared in Example 92 to give 0.3959 g (yield, 84.3 %) of the objective product.

Example 98

[Production of 1-allyl-3-(5-t-butyl-3-isoxazolyl)-2,4-dioxoimidazolidine]

In the same manner as in Example 8 (3) is treated 1.34 g of the product prepared in Example 92 to give 1.25 g (yield, 79.1 %) of the objective product as crystals. This is recrystallized from a mixture of hexane and ethyl

acetate to give colorless plates, m.p. 102.5 - 103.5°C.

Example 99

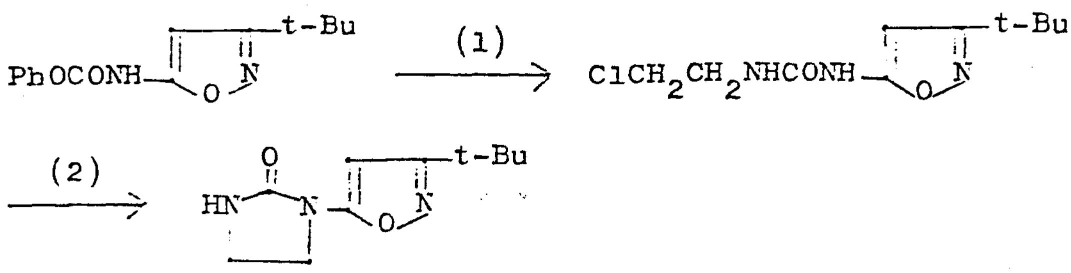


[Production of 1-(3-t-butyl-5-isoxazolyl)-2-imidazolidinone]

(1) To a solution of 7.81 g of phenyl 3-t-butylisoxazole-5-carbamate in 60 ml of methylene chloride are added 5.22 g of 2-chloroethylamine hydrochloride and 7.59 g of triethylamine, and the mixture is stirred overnight at room temperature and then concentrated in vacuo. The residue is purified by chromatography on silica gel to give 7.37 g of the product as crude crystals. This is recrystallized from a mixture of cyclohexane and ethyl acetate to give 1-(2-chloroethyl)-3-(3-t-butyl-5-isoxazolyl)urea as colorless needles, m.p. 126.0 - 127.0°C.

(2) To 4.91 g of the product prepared in the above (1) are added 50 ml of water and 5.61 g of 86 % aqueous potassium hydroxide, and the mixture is stirred at room temperature for 20 minutes. To the reaction mixture is added water, and the mixture is extracted with methylene chloride, dried over anhydrous sodium sulfate, and concentrated in vacuo to give 4.20 g of the product as crude crystals. This is

recrystallized from a mixture of cyclohexane and ethyl acetate to give 1-(3-t-butyl-5-isoxazolyl)-2-imidazolidinone as colorless prisms, m.p. 186.0 - 187.0°C.

Example 100 - 101

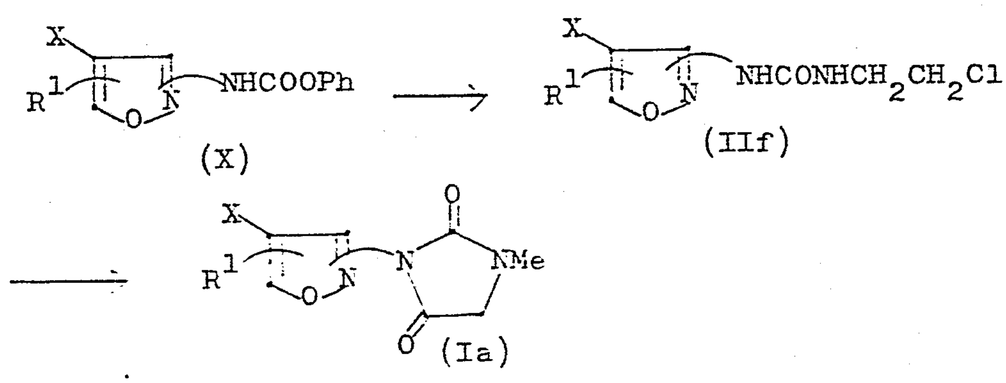

(X) (IIf) (Ia)

The starting material (X) is treated in the same manner as in Example 99 to give the objective product (Ia) as well as the intermediate (IIf). The results are summarized in Table 11.

Table 11

| | II f | | | | | I a | | |
|---|---|---|---|---|---|---|---|---|
| Example Nos. | $R^1$ | X | Amount of material (X) (g) | Yield (g) (%) | m.p. (°C) Appearance | Amount of IIf used (g) | Yield (g) (%) | m.p. (°C) Appearance |
| 100 | 5-i-Pr | H | 9.85 | 9.39<br>101.0* | 138.0 - 139.0<br>colorless needles | 4.63 | 3.82<br>97.8 | 148.0 - 149.0<br>colorless needles |
| 101 | 3-i-Pr | H | 9.85 | 9.37<br>101.1* | 94.5 - 95.5<br>colorless needles | 4.63 | 3.83<br>98.1 | 143.0 - 144.0<br>colorless prisms |

* Crude yield

Example 102

[Production of 1-methyl-3-(5-t-butyl-3-isoxazolyl)-4,5-dimethoxy-2-imidazolidinone]

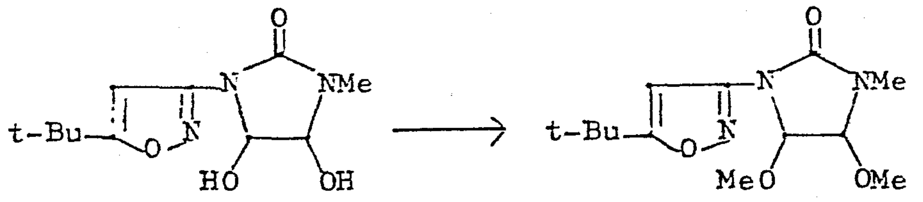

(mixture of cis-trans isomers)    (cis isomer or trans isomer)

To a solution of 1.79 g of 1-methyl-3-(5-t-butyl-3-isoxazolyl)-4,5-dihydroxy-2-imidazolidinone (cis : trans = 8:92) in dry tetrahydrofuran cooled to -78°C is added 1.62 g of 97 % potassium t-butoxide, and the mixture is stirred at -78°C for 30 minutes. The mixture is mixed with 2.19 g of methyl iodide and stirred at room temperature for additional 30 minutes. The reaction mixture is concentrated in vacuo, and the residue is mixed with water. The mixture is extracted with methylene chloride, and the methylene chloride layer is dried over anhydrous sodium sulfate and then concentrated. The residue is purified by chromatography on silica gel to give 1.75 g (yield, 88.1 %) of the trans isomer and 0.18 g (yield, 9.2 %) of the cis isomer as colorless oils, respectively.

trans isomer

NMR, $\delta^{CDCl_3}$ : 4.60(s, 1H), 5.21(s, 1H)

<u>cis isomer</u>

NMR, $\delta^{CDCl_3}$ : 4.82(d, 1H, J=6Hz), 5.40(d, 1H, J=6Hz)

<u>Example 103</u>

The following compounds are obtained in the same manner as in Example 1.

| | m.p. (°C) |
|---|---|
| 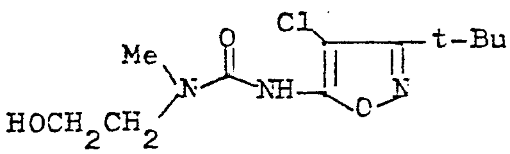 | 146.0 - 147.0 |
| 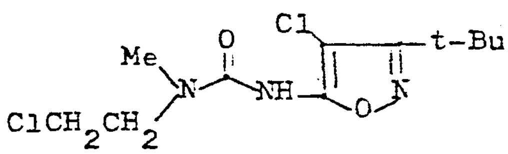 | 111.0 - 114.0 |
| 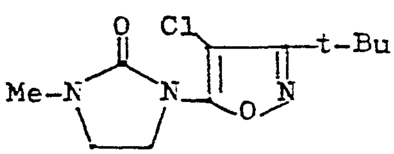 | 88.0 - 89.0 |

Example 104

[Production of 1,4-dimethyl-3-(5-t-butyl-3-isoxazolyl)-2-imidazolidinone]

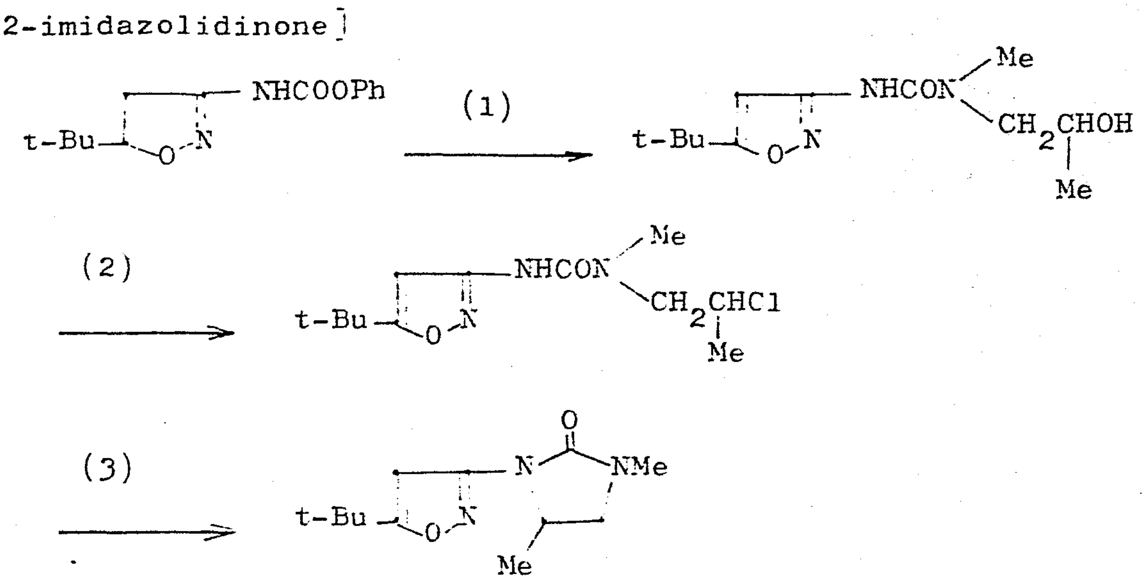


(1) To a solution of 3.90 g (15 mmol) of phenyl 5-t-butylisoxazole-3-carbamate in 25 ml of methylene chloride are added 1.67 g (19.2 mmol) of 96 % 1-methyl-2-methylaminoethanol and 1.52 g (15 mmol) of triethylamine with stirring. The mixture is allowed to react overnight at room temperature and then acidified with 80 ml of 2 % hydrochrolic acid. The reaction mixture is extracted twice with 80 ml of methylene chloride. The methylene chloride layer is dried over anhydrous sodium sulfate and evaporated. The residue is purified by chromatography on silica gel to give 3.75 g (yield: 97.9 %) of 1-methyl-1-(2-hydroxypropyl)-3-(5-t-butyl-3-isoxazolyl)urea as colorless crystals. This is recrystallized from n-hexane - benzene

to give the said compound as colorless prisms melting at 116 - 117°C.

(2) A mixture of 3.06 g (12.0 mmol) of the compound prepared in the above step (1), 24 ml of benzene. and 1.72 g (14.4 mmol) of thionyl chloride is refluxed with stirring for 10 minutes. The mixture is cooled and the precipitated crystals are collected by filtration. The filtrate is evaporated to remove benzene and the residue is purified by chromatography on silica gel to give 1.66 g (yield: 50.5 %) of 1-methyl-(2-chloropropyl)-3-(5-t-butyl-3-isoxazolyl)urea as slightly yelowish crystals.

Anal. Calcd. (%) (for $C_{12}H_{20}N_3O_2Cl$):

C, 52.64; H, 7.38; N, 15.35; Cl, 12.95

Found (%): C, 52.18; H, 7.21; N, 15.28; Cl, 12.89 .

(3) To a mixture of 1.26 g (4.6 mmol) of the product prepared in the above step (2) and 1.5 ml of pyridine is added a solution of 1.29 g (23.0 mmol) of potassium hydroxide in 14 ml of water. The mixture is allowed to react for 2 hours, and 50 ml of water is added thereto. The reaction mixture is acidified with conc. hydrochloric acid to adjust to lower than pH 2 and extracted twice with 80 ml of methylene chloride. The methylene chloride layer is dried over anhydrous sodium sulfate and evaporated. The resulting residue is purified by chromatography on

silica gel to give 0.96 g (yield: 87.9 %) of 1,4-dimethyl-3-(5-t-butyl-3-isoxazolyl)-2-imidazolidinone. This is recrystallized from n-hexane to give the said compound as colorless prisms.

m.p.: 62 - 63°C.

Example 105

[Production of 1,5-dimethyl-3-(5-t-butyl-3-isoxazolyl)-2-imidazolidinone]

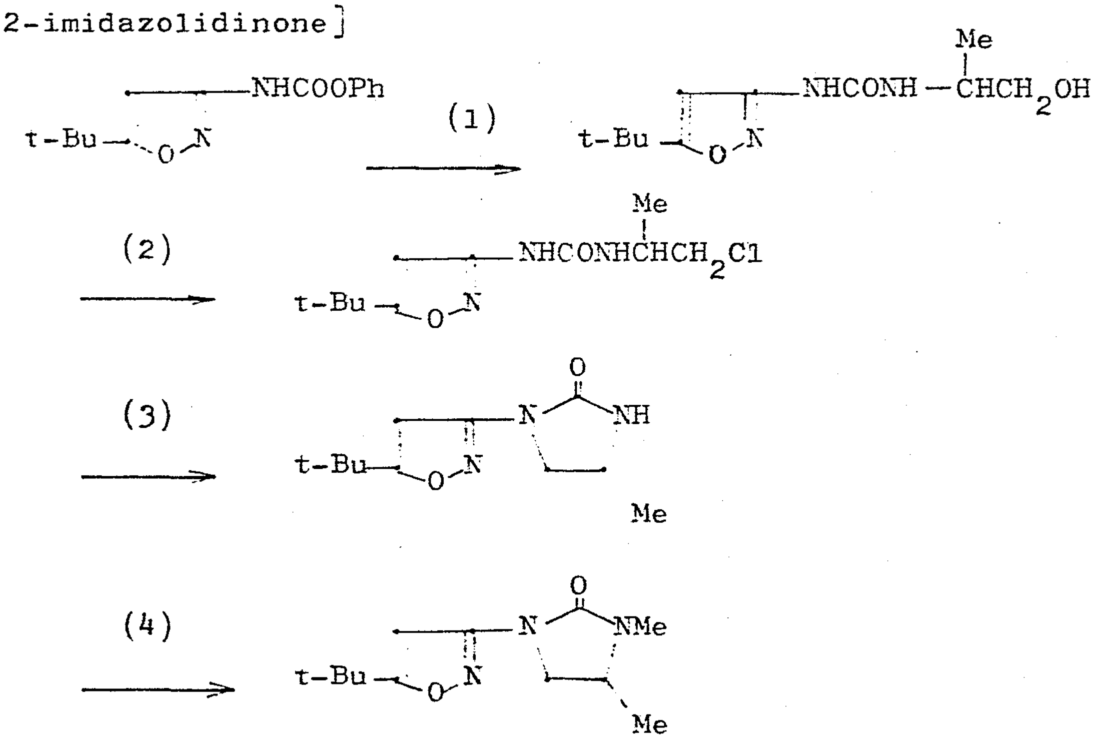


(1) To a solution of 3.90 g (15.0 mmol) of phenyl 5-t-butylisoxazole-3-carbamate in 25 ml of methylene chloride are added 1.35 g (18.0 mmol) of 2-aminopropanol and 1.52 g (16.5 mmol) of triethylamine with stirring. The mixture is allowed to react overnight at room temperature and then acidified with 80 ml of 2 % hydrochloric acid.

The reaction mixture is extracted twice with 80 ml of methylene chloride. The methylene chloride layer is dried over anhydrous sodium sulfate and evaporated. The residue is purified by chromatography on silica gel to give 3.33 g (yield: 92.0 %) of 1-(1-hydroxymethylethyl)-3-(5-t-butyl-3-isoxazolyl)urea as slightly yellowish crystals.

Anal. Calcd.(%):(for $C_{11}H_{19}N_3O_3$):

C, 54.75; H, 7.94; N, 17.42

Found (%): C, 54.10; H, 7.85; N, 17.14.

(2) A mixture of 2.90 g (12.0 mmol) of the compound prepared in the above step (1), 24 ml of benzene, and 1.72 g (14.4 mmol) of thionyl chloride is refluxed with stirring for 10 minutes. The mixture is evaporated under reduced pressure. The residue is purified by chromatography on silica gel to give 2.88 g (yield: 92.4 %) of 1-(1-chloromethylethyl)-3-(5-t-butyl-3-isoxazolyl)urea. This is recrystallized from benzene - n-hexane to give colorless plates. m.p.: 110 - 111°C.

Anal. Calcd.(%) (for $C_{11}H_{18}N_3O_2Cl$):

C, 50.86; H, 7.00; N, 16.18; Cl, 13.65

Found (%): C, 51.13; H, 6.93; N, 16.03; Cl, 13.66.

(3) To a mixture of 2.34 g (9.0 mmol) of the product prepared in the above step (2) and 3 ml of pyridine is added a solution of 2.52 g (45.0 mmol) of potassium

hydroxide in 27 ml of water. The mixture is allowed to react for 2 hours, and 100 ml of water is added thereto. The reaction mixture is acidified with conc, hydrochloric acid to adjust to lower than pH 2 and extracted twice with 100 ml of methylene chloride. The methylene chloride layer is washed with 100 ml of saturated sodium chloride aqueous solution, dried over anhydrous sodium sulfate, and evaporated. The residue is recrystallized from ethylacetate - cyclohexane to give 1.33 g (yield: 66.2 %) of 3-(5-t-butyl-3-isoxazolyl)-5-methyl-2-imidazolidinone as colorless plates, and 0.37 g (yield: 18.4 %) of the said compound is recovered from the mother liquor by chromatography on silica gel.

m.p.: 138 - 139°C.

(4) To a mixture of 1.12 g (5.0 mmol) of the product provided in the above step (3) and 20 ml of dry dimethylformamide is added 0.22 g (5.5 mmol) of 60 % sodium hydride with stirring at a temperature of lower than 20°C for 30 minutes. To the mixture is added a solution of 0.92 g (6.0 mmol) of methyliodide dissolved in 3 ml of dry dimethylformamide over 5 minutes. The mixture is allowed to react for 1 hour at room temperature. The reaction mixture is evaporated under reduced pressure, mixed with 80 ml of water, and extracted twice with 80 ml of methylene chloride. The methylene chloride layer is

dried over anhydrous sodium sulfate and evaporated. The residue is purified by chromatography on silica gel to give 1.13 g (yield: 95.2 %) of 1,5-dimethyl-3-(5-t-butyl-3-isoxazolyl)-2-imidazolidinone. This is recrystallized from n-hexane to give colorless prisms.

m.p.: 74 - 75°C.

In the following section of Referential Example, processes for the production of the starting materials are shown.

Referential Example 1

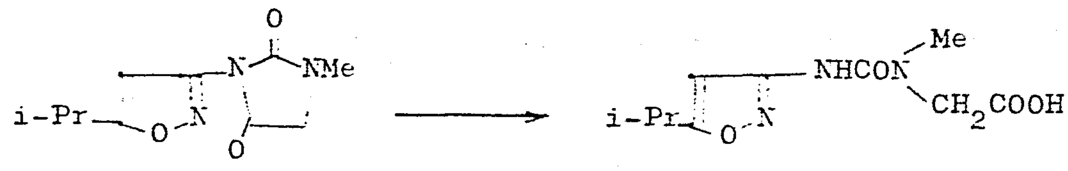

[Production of 1-methyl-1-carboxymethyl-3-(5-isopropyl-3-isoxazolyl)urea]

To 15.87g of the product prepared in Example 89 is added 62.56 g of 5 % aqueous sodium hydroxide, and the mixture is stirred at room temperature for an hour, adjusted to pH 1 with 36 % hydrochloric acid, and then extracted with chloroform. The chloroform layer is dried over anhydrous sodium sulfate and concentrated in vacuo. The residue is dispersed in benzene to give crystals, which are collected by filtration to give 16.91 g (yield: 98.6 %) of the objective product as crystals, m.p. 133.0 - 133.5°C (dec.).

Referential Examples 2 - 5

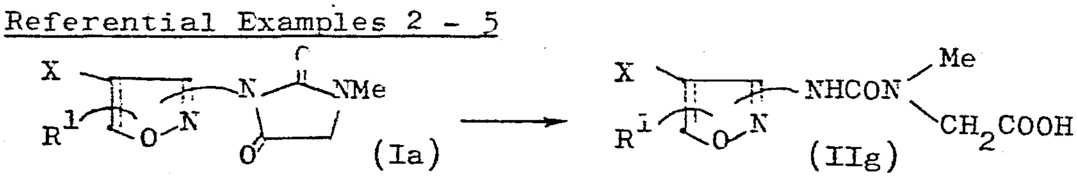

(Ia) (IIg)

The starting materials (Ia) is treated in the same manner as in Referential Example 1 to give the objective product (IIg). The results are summarized in Table 12.

Table 12

| Ref. Ex. Nos. | $R^1$ | X | Amount of starting material (Ia) (g) | Yield (g) | Yield (%) | m.p. (°C) | Appearance |
|---|---|---|---|---|---|---|---|
| 2 | 5-t-Bu | H | 5.16 | 5.53 | 99.8 | 140.5-141.0(dec.) | colorless needles |
| 3 | " | Cl | 3.30 | 3.01 | 85.9 | 114.0-114.5( " ) | colorless prisms |
| 4 | 3-t-Bu | H | 2.18 | 2.11 | 90.0 | 130.5-131.0( " ) | colorless powdery crystals |
| 5 | 3-i-Pr | H | 7.29 | 7.75 | 98.2 | 133.0-133.5( " ) | colorless prisms |

Referential Example 6

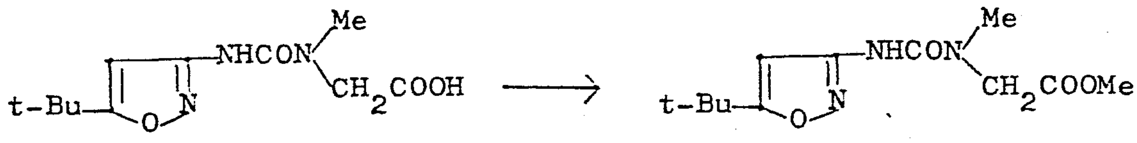

[Production of 1-methyl-1-methoxycarbonylmethyl-3-(5-t-butyl-3-isoxazolyl)urea]

To a mixture of 9 ml of ethyl ether with 2.30 g of the product prepared in Ref. Example2 is gradually added an ethereal solution of diazomethane under ice-cooling until the yellow of diazomethane remains. After completion of the addition the mixture is stirred for 15 minutes and acetic acid is then added to deactivate the diazomethane remaining unchanged. Water is added to the mixture, which is extracted with methylene chloride. The methylene chloride layer is dried over anhydrous sodium sulfate and concentrated in vacuo. To a solution of the residue in a small amount of methylene chloride is added cyclohexane, and the precipitated crystals of the product are collected by filtration. Yield, 1.65 g (yield, 68.1 %). m.p. 120.5 - 122.5°C, colorless prisms.

Referential Examples 7 - 10

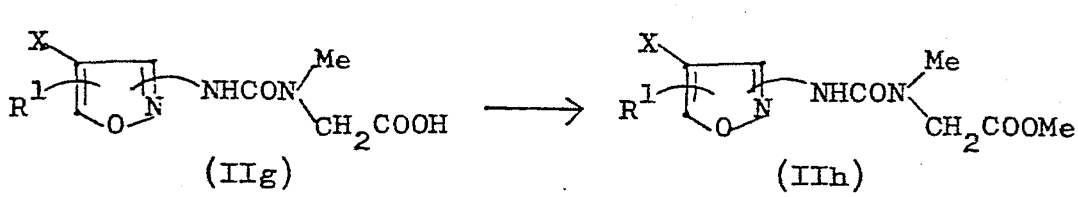

(IIg) (IIh)

The starting material (IIg) is treated in the same

manner as in Ref. Example6 to give the objective product (IIh). The results are summarized in Table 13.

Table 13

| Ref. Ex. Nos. | $R^1$ | X | Amount of starting material IIg (g) | Yield (g) | Yield (%) | m.p. (°C) | Appearance |
|---|---|---|---|---|---|---|---|
| 7 | 5-t-Bu | Cl | 1.14 | 1.05 | 87.7 | 118.5 - 119.5 | colorless powdery crystals |
| 8 | 5-i-Pr | H | 2.17 | 1.91 | 83.1 | 105.0 - 107.0 | colorless needles |
| 9 | 3-t-Bu | H | 1.08 | 1.06 | 93.1 | 70.5 - 72.5 | " |
| 10 | 3-i-Pr | H | 2.41 | 2.15 | 82.3 | 71.0 - 73.0 | " |

Referential Example 11

[Production of 1-methoxycarbonylmethyl-3-(5-t-butyl-3-isoxazolyl)urea] (Alternative route)

To 6.65 g of 5-t-butyl-3-isoxazolyl cyanate are added 80 ml of dry benzene and 3.56 g of glycine methyl ester, and the mixture is refluxed for three hours and then concentrated in vacuo. The residue is purified by chromatography on silica gel to give 7.86 g (yield, 77.0 %) of the objective product as crystals.

Referential Example 12

[Production of 1-carboxymethyl-3-(5-t-butyl-3-isoxazolyl)-urea] (Alternative route)

To 10.41 g of phenyl 5-t-butylisoxazole-3-carbamate are added 100 ml of dry pyridine and 6.01 g of glycine, and the mixture is stirred at 70°C for 48 hours. The reaction mixture is concentrated in vacuo, 100ml of 8% aqueous sodium bicarbonate added to the residue, and the mixture extracted with methylene chloride to remove methylene chloride-soluble materials. To the aqueous layer is gradually added 36 % hydrochloric acid to adjust at pH 1.

The mixture is then ice-cooled and the precipitated crystals are collected by filtration, and dried in vacuo at 60°C for 16 hours, whereby 9.52 g (yield, 98.7 %) of the objective product is obtained as crystals.

Referential Example 13

[Production of 1-carboxymethyl-3-(3-t-butyl-5-isoxazolyl)-urea] (Alternative route)

In the same manner as in Ref. Example 12, 14.56 g of phenyl 3-t-butylisoxazole-5-carbamate is treated to give 8.78 g (yield, 72.8 %) of the objective product as crystals.

Referential Examples 14 - 17

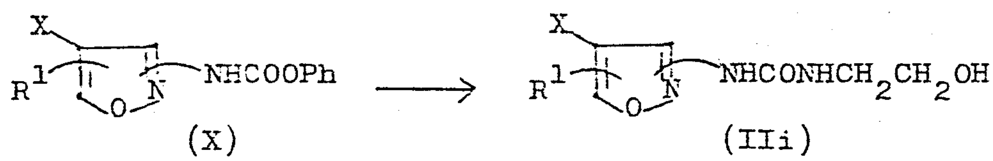

(X) (IIi)

To a solution of the starting material (X) in methylene chloride is added ethanolamine (2 mol eq.), and the mixture is stirred overnight at room temperature. The reaction mixture is then concentrated in vacuo, and the residue is purified by chromatography on silica gel to give the product (IIi). The results are summarized in Table 14.

Table 14

| Ref. Ex. Nos. | $R^1$ | X | Amount of starting material (X) (g) | Yield (g) | Yield (%) | m.p. (°C)/IR($CHCl_3$) $cm^{-1}$ Appearance |
|---|---|---|---|---|---|---|
| 14 | 5-t-Bu | Cl | 14.74 | 11.16 | 85.3 | 137.5 - 139.0 colorless plates |
| 15 | 5-i-Pr | H | 2.46 | 1.99 | 93.3 | 1680, 1615, 1560 pale-yellow oil |
| 16 | 3-t-Bu | " | 2.60 | 2.28 | 100.3* | 126.0 - 127.0 colorless needles |
| 17 | 3-i-Pr | " | 12.31 | 13.08 | 101.7* | 76.0 - 77.0 pale-yellow plates |

* Crude yield

Rerefential Example 18

[Production of 1-(3-hydroxypropyl)-3-(5-t-butyl-4-chloro-3-isoxazolyl)urea]

In the same manner as in Example 12 (1) is treated 2.95 g of phenyl 5-t-butyl-4-chloroisoxazole-3-carbamate to give 2.41 g (yield, 87.4 %) of the objective product as a pale-yellow oil. IR ($CHCl_3$) : 1687, 1555, 1530 $cm^{-1}$.

Referential Example 19

a) 1-(5-t-butyl-3-isoxazolyl)-5-hydroxy-2-imidazolidinone: colorless plates, m.p. 179 - 181°C (dec.).

b) 3-(5-t-butyl-3-isoxazolyl)-4-methoxy-1-methyl-2-imidazolidinone: colorless oil, IR ($CHCl_3$) : 1720, 1598, 1435 $cm^{-1}$.

c) 4-allyloxy-3-(5-t-butyl-3-isoxazolyl)-1-methyl-2-imidazolidinone: colorless oil, IR ($CHCl_3$) : 1722, 1599, 1440 $cm^{-1}$.

The isoxazole cyclic ureas (I) of the present invention show a potent herbicidal activity against a variety of weeds at a small application rate. They have an excellent herbicidal activity against general weeds, e.g.

Digitaria adscendens (large crabgrass),
Setaria viridis (green foxtail),
Echinochloa crusgalli (barnyard grass),
Amaranthus viridis (green amaranth),
Chenopodium album (smooth pigweed),

Cyperus micro-iria (flat-sedge),

Mollugo stricta (carpet-weed),

Stellaria media (common chickweed),

Segina japonica (pearlwort),

Stellaria Alsine (bog-stichwort),

Capsella Bursa-pastoris (Shepherd's purse),

Alopecurus aequalis (water foxtail),

Polygonum longisetum (smartweed),

Trigonotis peduncularis (-),

Gnaphalium affine (cottonweed),

Echinochloa oryzicola (barnyard grass),

Monchoria vaginalis (monochoria),

Cyperus difformis (umbrella plant),

Rotala indica (tooth cup), and

Dopatrium junceum (-).

In more detail, at an application rate of 1.25 - 20 g/a they are practically nontoxic to common field crops, such as corn, sugar cane, sorghum, wheat, soybean, cotton and tomato, or the harmful influence over other crop plants, if any, is so little that they could easily recover from the damage. The compounds (I) of the present invention may be applied as a selective or non-selective herbicide for crop lands, e.g. plowed fields, orchards, tea plantations, mulberry fields, fallow lands and pastures; or non-crop lands, e.g., railbeds, roads, lawns, factory sites, dry riverbeds,

residential quarters, park green districts, forest lands, prepared lands and vacant lands.

Furthermore, the compounds (I) of the present invention are harmless and safe to the human, domestic animals, and birds or poultry, and show a very low toxicity to fishes, shells and the like. Consequently, the herbicides of this invention are quite safe ones and therefore there is no problem about the residual toxicity in soil.

The present compounds (I) each may be used in combination with known herbicides such as diuron, atrazine, prometryn, alachlor and trifluralin for the purpose of extending its herbicidal spectrum and getting an additional or synergistic herbicidal effect.

The herbicides of the present invention may be applied over the soil such as sandy soil and clay soil as well as the ordinary soil such as sandy loam soil and loamy soil. They may be applied even to one of clay soil, i.e., montmorillonite soil which occasionally has an influence on the occurrence of the herbicidal activity.

The herbicides of the present invention, i.e. cyclic ureas of isoxazole (I), may suitably be used in combination with a customary solid or liquid carrier or carriers, if desired, together with additional adjuvants (e.g. emulsifiers, stabilizers, dispersants, suspending agents, spreaders, penetrants, wetting agents) in order to formulate

more refined formulations (e.g. emulsions, wettable powders, granules, dusts, pellets).

Representatives of the carriers are solid carriers (e.g. clay, talc, diatomaceous earth, bentonite), liquid carriers (e.g. water, alcohols, acetone, benzene, toluene, xylene, solvent naphtha, cyclohexane) and the like.

The herbicide formulations of this invention may be used in combination with other agricultural chemicals (e.g. insecticides, fungicides or other herbicides), manuring ingredients (e.g. ammonium sulfate, urea) or soil treating agents.

The activities of the present herbicides are illustrated in the following Experiments.

The test-compound Nos. correspond to the respective numbers of Examples, and additionally the following compounds (test-compound Nos. 106 - 126) were used in the test.

| Test-compound No. | Name of compound |
|---|---|
| 106 | 1-(2-hydroxyethyl)-3-(5-t-butyl-3-isoxazolyl)urea |
| 107 | 1-methyl-1-(2-hydroxyethyl)-3-(5-t-butyl-3-isoxazolyl)urea |
| 108 | 1-methyl-1-(2-hydroxyethyl)-3-(5-t-butyl-4-chloro-3-isoxazolyl)urea |
| 109 | 1-methyl-1-(2-hydroxyethyl)-3-(5-isopropyl-3-isoxazolyl)urea |
| 110 | 1-methyl-1-(2-hydroxyethyl)-3-(3-t-butyl-5-isoxazolyl)urea |
| 111 | 1-methyl-1-(2-hydroxyethyl)-3-(3-t-butyl-4-chloro-5-isoxazolyl)urea |
| 112 | 1-methyl-1-(2-hydroxyethyl)-3-(3-isopropyl-5-isoxazolyl)urea |
| 113 | 1-methyl-1-(2-chloroethyl)-3-(5-t-butyl-3-isoxazolyl)urea |
| 114 | 1-methyl-1-(2-chloroethyl)-3-(5-t-butyl-4-chloro-3-isoxazolyl)urea |
| 115 | 1-methyl-1-(2-chloroethyl)-3-(5-isopropyl-3-isoxazolyl)urea |
| 116 | 1-methyl-1-(2-chloroethyl)-3-(3-t-butyl-5-isoxazolyl)urea |
| 117 | 1-methyl-1-(2-chloroethyl)-3-(3-isopropyl-5-isoxazolyl)urea |
| 118 | 1-methyl-1-(3-hydroxypropyl)-3-(5-t-butyl-3-isoxazolyl)urea |
| 119 | 1-methyl-1-(3-chloropropyl)-3-(5-t-butyl-3-isoxazolyl)urea |
| 120 | 1-methyl-1-carboxymethyl-3-(5-t-butyl-3-isoxazolyl)urea |

| Test-compound Nos. | Name of compound |
|---|---|
| 121 | 1-methyl-1-carboxymethyl-3-(5-t-butyl-4-chloro-3-isoxazolyl)urea |
| 122 | 1-methyl-1-methoxycarbonylmethyl-3-(5-t-butyl-3-isoxazolyl)urea |
| 123 | 1-methyl-1-methoxycarbonylmethyl-3-(5-t-butyl-4-chloro-3-isoxazolyl)urea |
| 124 | 1-methyl-1-(2-hydroxypropyl)-3-(5-tert-butyl-3-isoxazolyl)urea |
| 125 | 1-methyl-1-(2-chloropropyl)-3-(5-tert-butyl-3-isoxazolyl)urea |
| 126 | 1-(1-hydroxymethylethyl)-3-(5-tert-butyl-3-isoxazolyl)urea |

The compounds used as a reference are as follows:

Reference compound 1

Diuron: 1,1-dimethyl-3-(3,4-dichlorophenyl)urea

(U. S. Pat. No. 2,768,971)

Reference compound 2

Linuron: 1-methoxy-1-methyl-3-(3,4-dichlorophenyl)urea

(U. S. Pat. No. 2,960,534)

<u>Experiment 1</u>

a) Test method

1. Pre-emergence test

Seeds of a test plant were sowed on the sandy loam charged in a square pot (7.1 x 7.1 cm) made of vinyl chloride. After being sowed, the seeds were covered with

the sandy loam and an aqueous suspension (Tween 20 is used at a concentration of 100 ppm as a spreader.) of the test compound is applied over the surface of the sandy loam.

The suspension of the test compound was prepared by dilution with water at a concentration of 10g/10L or 30g/10L and applied at a rate of 10L/are with an automatic metal sprayer.

The pots were kept at a temperature of 25°C in a greenhouse under the natural sunlight. The survival rate is evaluated three weeks after the application of the chemicals.

2. Post-emergence test

Seeds of a test plant were sowed on the sandy loam charged in a square pot (7.1 x 7.1 cm) of vinyl chloride. After being sowed, the seeds were covered with the sandy loam and then grew for ten days. Tween 20 was used at a concentration of 100 ppm as a spreader. The aqueous suspension of the test compound was applied over the young plants ten days after the sowing. The suspension of the test compound was prepared by dilution with water at a concentration of 10g/10L or 30g/10L and applied at a rate of 10L/are with an automatic metal sprayer. The pots were kept at a temperature of 25°C in a greenhouse under the natural sunlight. The survival rate was evaluated three weeks after the application.

b) Method of evaluation

The outward change after the application of the chemicals was checked by eye observation at prefixed intervals and the survival rate was calculated from the number of the survival plants after three weeks.

The evaluation was made on the following six degree of score.

| Degree of the evaluation | Score |
|---|---|
| Survival rate: 0 - 10 % | 5 |
| " 11 - 25 % | 4 |
| " 26 - 50 % | 3 |
| " 51 - 75 % | 2 |
| " 76 - 90 % | 1 |
| " 91 -100 % | 0 |

c) Result

Results in the herbicidal test are summarized in Table 15. The abbreviation used for the test plant in Table 15 has the following meaning.

A: wheat
B: barnyard grass
C: large crabgrass
D: oilseed rape
E: smartweed
F: spleen amaranth

Table 15

| Compound Nos. | Amount (g/a) | Pre-emergence | | | | | | Post-emergence | | | | | |
|---|---|---|---|---|---|---|---|---|---|---|---|---|---|
| | | A | B | C | D | E | F | A | B | C | D | E | F |
| 1 | 30 | 1 | 5 | 5 | 5 | 5 | 5 | 2 | 5 | 5 | 5 | 5 | 5 |
| | 10 | 0 | 4 | 5 | 4 | 5 | 5 | 0 | 5 | 5 | 5 | 5 | 5 |
| 2 | 30 | 1 | 5 | 5 | 5 | 5 | 5 | 1 | 5 | 5 | 5 | 5 | 5 |
| | 10 | 1 | 5 | 5 | 5 | 5 | 5 | 1 | 5 | 5 | 5 | 5 | 5 |
| 3 | 30 | 0 | 5 | 5 | 5 | 5 | 5 | 0 | 5 | 5 | 5 | 5 | 5 |
| | 10 | 0 | 3 | 4 | 4 | 5 | 3 | 0 | 3 | 5 | 5 | 5 | 5 |
| 4 | 30 | 0 | 5 | 5 | 5 | 5 | 5 | 0 | 5 | 5 | 5 | 5 | 5 |
| | 10 | 0 | 3 | 2 | 5 | 5 | 5 | 0 | 2 | 5 | 5 | 5 | 5 |
| 5 | 30 | 0 | 3 | 5 | 5 | 5 | 5 | 0 | 3 | 5 | 5 | 5 | 5 |
| | 10 | 0 | 1 | 1 | 4 | 3 | 5 | 0 | 1 | 5 | 5 | 5 | 5 |
| 7 | 30 | 1 | 3 | 5 | 3 | 4 | 4 | 1 | 5 | 5 | 1 | 5 | 5 |
| | 10 | 1 | 0 | 3 | 0 | 0 | 1 | 1 | 1 | 2 | 3 | 5 | 5 |
| 8 | 30 | 0 | 4 | 5 | 5 | 3 | 5 | 0 | 0 | 2 | 5 | 5 | 5 |
| | 10 | 0 | 1 | 1 | 1 | 2 | 5 | 0 | 1 | 2 | 5 | 3 | 5 |
| 10 | 30 | 4 | 5 | 5 | 5 | 5 | 5 | 5 | 5 | 5 | 5 | 5 | 5 |
| | 10 | 2 | 5 | 5 | 5 | 5 | 5 | 3 | 5 | 5 | 5 | 5 | 5 |
| 11 | 30 | 0 | 4 | 3 | 5 | 4 | 5 | 1 | 1 | 4 | 5 | 4 | 5 |
| | 10 | 0 | 1 | 1 | 4 | 3 | 5 | 0 | 1 | 2 | 5 | 3 | 4 |

| Compound Nos. | Amount (g/a) | Pre-emergence | | | | | | Post-emergence | | | | | |
|---|---|---|---|---|---|---|---|---|---|---|---|---|---|
| | | A | B | C | D | E | F | A | B | C | D | E | F |
| 14 | 30 | 5 | 5 | 5 | 5 | 5 | 5 | 5 | 5 | 5 | 5 | 5 | 5 |
| | 10 | 1 | 2 | 5 | 5 | 5 | 5 | 2 | 5 | 5 | 5 | 5 | 5 |
| 15 | 30 | 0 | 2 | 5 | 5 | 5 | 5 | 0 | 1 | 2 | 5 | 5 | 5 |
| | 10 | 0 | 0 | 0 | 1 | 2 | 2 | 0 | 1 | 1 | 5 | 1 | 4 |
| 17 | 30 | 1 | 3 | 5 | 5 | 3 | 5 | 2 | 3 | 3 | 5 | 5 | 5 |
| | 10 | 0 | 2 | 3 | 2 | 3 | 3 | 0 | 1 | 1 | 5 | 1 | 2 |
| 18 | 30 | 0 | 2 | 4 | 5 | 5 | 5 | 1 | 2 | 4 | 5 | 5 | 5 |
| | 10 | 0 | 2 | 4 | 5 | 4 | 5 | 1 | 1 | 5 | 5 | 5 | 5 |
| 19 | 30 | 0 | 0 | 3 | 0 | 3 | 5 | 0 | 1 | 3 | 5 | 5 | 5 |
| | 10 | 0 | 0 | 0 | 0 | 0 | 3 | 0 | 0 | 1 | 2 | 2 | 2 |
| 21 | 30 | 0 | 1 | 1 | 1 | 0 | 4 | 0 | 1 | 2 | 5 | 1 | 3 |
| | 10 | 0 | 0 | 0 | 1 | 2 | 2 | 0 | 1 | 1 | 4 | 1 | 2 |
| 26 | 30 | 3 | 5 | 5 | 5 | 5 | 5 | 4 | 5 | 5 | 5 | 5 | 5 |
| | 10 | 0 | 4 | 5 | 5 | 5 | 5 | 1 | 3 | 5 | 5 | 5 | 5 |
| 28 | 30 | 1 | 1 | 5 | 3 | 3 | 5 | 1 | 1 | 2 | 5 | 5 | 5 |
| | 10 | 0 | 0 | 3 | 5 | 2 | 5 | 1 | 2 | 1 | 5 | 3 | 5 |
| 29 | 30 | 0 | 0 | 1 | 5 | 1 | 5 | 1 | 1 | 3 | 5 | 5 | 5 |
| | 10 | 0 | 0 | 1 | 5 | 1 | 5 | 1 | 1 | 1 | 5 | 2 | 5 |
| 30 | 30 | 0 | 4 | 3 | 0 | 5 | 5 | 0 | 5 | 5 | 5 | 5 | 5 |
| | 10 | 0 | 2 | 1 | 0 | 1 | 3 | 0 | 1 | 1 | 4 | 5 | 5 |

| Compound Nos. | Amount (g/a) | Pre-emergence | | | | | | Post-emergence | | | | | |
|---|---|---|---|---|---|---|---|---|---|---|---|---|---|
| | | A | B | C | D | E | F | A | B | C | D | E | F |
| 31 | 30 | 1 | 5 | 5 | 5 | 5 | 5 | 1 | 3 | 5 | 5 | 5 | 5 |
| | 10 | 0 | 1 | 2 | 1 | 4 | 5 | 0 | 1 | 4 | 5 | 4 | 5 |
| 32 | 30 | 3 | 5 | 5 | 5 | 5 | 5 | 4 | 5 | 5 | 5 | 5 | 5 |
| | 10 | 1 | 3 | 3 | 5 | 5 | 5 | 1 | 4 | 1 | 5 | 5 | 5 |
| 40 | 30 | 0 | 4 | 5 | 5 | 5 | 5 | 1 | 5 | 5 | 5 | 5 | 5 |
| | 10 | 0 | 0 | 5 | 3 | 3 | 5 | 1 | 5 | 5 | 5 | 5 | 5 |
| 41 | 30 | 1 | 4 | 4 | 5 | 5 | 5 | 1 | 1 | 5 | 5 | 5 | 5 |
| | 10 | 0 | 1 | 1 | 1 | 3 | 5 | 1 | 1 | 3 | 5 | 4 | 5 |
| 42 | 30 | 1 | 1 | 2 | 5 | 4 | 5 | 1 | 1 | 2 | 5 | 5 | 5 |
| | 10 | 0 | 1 | 2 | 4 | 3 | 5 | 0 | 1 | 1 | 3 | 1 | 5 |
| 44 | 30 | 2 | 5 | 5 | 5 | 5 | 5 | 4 | 5 | 5 | 5 | 5 | 5 |
| | 10 | 1 | 4 | 5 | 5 | 5 | 5 | 1 | 5 | 5 | 5 | 5 | 5 |
| 45 | 30 | 1 | 5 | 4 | 5 | 5 | 5 | 1 | 1 | 4 | 5 | 5 | 5 |
| | 10 | 0 | 1 | 1 | 2 | 1 | 5 | 0 | 0 | 3 | 5 | 4 | 5 |
| 46 | 30 | 0 | 1 | 2 | 3 | 3 | 5 | 0 | 1 | 3 | 5 | 5 | 5 |
| | 10 | 0 | 0 | 1 | 1 | 1 | 5 | 0 | 1 | 2 | 5 | 1 | 5 |
| 47 | 30 | 1 | 1 | 3 | 5 | 5 | 5 | 1 | 1 | 1 | 5 | 2 | 5 |
| | 10 | 0 | 0 | 1 | 4 | 1 | 5 | 0 | 0 | 1 | 1 | 1 | 5 |
| 49 | 30 | 0 | 1 | 3 | 2 | 4 | 3 | 0 | 4 | 5 | 5 | 5 | 5 |
| | 10 | 0 | 2 | 4 | 3 | 3 | 4 | 0 | 4 | 5 | 5 | 5 | 5 |

| Compound Nos. | Amount (g/a) | Pre-emergence | | | | | | Post-emergence | | | | | |
|---|---|---|---|---|---|---|---|---|---|---|---|---|---|
| | | A | B | C | D | E | F | A | B | C | D | E | F |
| 52 | 30 | 0 | 3 | 1 | 5 | 2 | 5 | 0 | 1 | 2 | 5 | 2 | 5 |
| | 10 | 0 | 0 | 1 | 1 | 1 | 5 | 0 | 0 | 1 | 5 | 1 | 3 |
| 58 | 30 | 0 | 4 | 5 | 1 | 2 | 5 | 1 | 5 | 5 | 4 | 4 | 5 |
| | 10 | 0 | 1 | 1 | 0 | 0 | 5 | 0 | 2 | 4 | 3 | 3 | 5 |
| 60 | 30 | 0 | 5 | 5 | 1 | 3 | 5 | 1 | 5 | 5 | 5 | 5 | 5 |
| | 10 | 0 | 0 | 0 | 0 | 0 | 5 | 0 | 2 | 1 | 4 | 2 | 5 |
| 61 | 30 | 1 | 5 | 5 | 5 | 5 | 5 | 1 | 5 | 5 | 5 | 5 | 5 |
| | 10 | 0 | 4 | 4 | 5 | 5 | 5 | 1 | 1 | 4 | 5 | 5 | 5 |
| 62 | 30 | 5 | 5 | 5 | 5 | 5 | 5 | 5 | 5 | 5 | 5 | 5 | 5 |
| | 10 | 2 | 5 | 5 | 5 | 5 | 5 | 3 | 4 | 2 | 5 | 5 | 5 |
| 63 | 30 | 3 | 5 | 5 | 5 | 5 | 5 | 5 | 5 | 5 | 5 | 5 | 5 |
| | 10 | 3 | 5 | 5 | 5 | 5 | 5 | 3 | 5 | 5 | 5 | 5 | 5 |
| 64 | 30 | 2 | 5 | 5 | 5 | 5 | 5 | 5 | 5 | 5 | 5 | 5 | 5 |
| | 10 | 0 | 4 | 2 | 5 | 5 | 5 | 1 | 1 | 4 | 5 | 5 | 5 |
| 65 | 30 | 0 | 5 | 2 | 5 | 5 | 5 | 1 | 5 | 5 | 5 | 5 | 5 |
| | 10 | 0 | 3 | 1 | 1 | 3 | 5 | 1 | 2 | 1 | 5 | 5 | 5 |
| 66 | 30 | 0 | 5 | 5 | 5 | 5 | 5 | 1 | 1 | 4 | 5 | 5 | 5 |
| | 10 | 0 | 5 | 5 | 5 | 5 | 5 | 1 | 1 | 3 | 5 | 5 | 5 |
| 67 | 30 | 0 | 5 | 5 | 5 | 5 | 5 | 1 | 1 | 5 | 5 | 5 | 5 |
| | 10 | 0 | 1 | 1 | 2 | 2 | 5 | 0 | 1 | 4 | 5 | 4 | 5 |

| Compound Nos. | Amount (g/a) | Pre-emergence | | | | | | Post-emergence | | | | | |
|---|---|---|---|---|---|---|---|---|---|---|---|---|---|
| | | A | B | C | D | E | F | A | B | C | D | E | F |
| 68 | 30 | 0 | 4 | 4 | 1 | 0 | 5 | 0 | 3 | 5 | 5 | 5 | 5 |
| | 10 | 0 | 1 | 1 | 1 | 0 | 5 | 0 | 1 | 4 | 5 | 5 | 5 |
| 69 | 30 | 0 | 0 | 3 | 2 | 2 | 5 | 0 | 1 | 2 | 5 | 5 | 5 |
| | 10 | 0 | 0 | 2 | 0 | 0 | 2 | 0 | 0 | 4 | 5 | 5 | 5 |
| 71 | 30 | 1 | 4 | 4 | 3 | 3 | 5 | 1 | 2 | 1 | 4 | 3 | 5 |
| | 10 | 0 | 1 | 2 | 1 | 1 | 5 | 0 | 0 | 0 | 1 | 1 | 4 |
| 73 | 30 | 5 | 5 | 5 | 5 | 5 | 5 | 5 | 5 | 5 | 5 | 5 | 5 |
| | 10 | 4 | 5 | 5 | 5 | 5 | 5 | 5 | 5 | 5 | 5 | 5 | 5 |
| 75 | 30 | 2 | 5 | 5 | 5 | 5 | 5 | 5 | 5 | 5 | 5 | 5 | 5 |
| | 10 | 2 | 5 | 5 | 5 | 5 | 5 | 3 | 5 | 5 | 5 | 5 | 5 |
| 76 | 30 | 5 | 5 | 5 | 5 | 5 | 5 | 5 | 5 | 5 | 5 | 5 | 5 |
| | 10 | 0 | 5 | 3 | 5 | 5 | 5 | 5 | 5 | 3 | 5 | 5 | 5 |
| 77 | 30 | 4 | 5 | 5 | 5 | 5 | 5 | 5 | 5 | 5 | 5 | 5 | 5 |
| | 10 | 0 | 5 | 3 | 5 | 5 | 5 | 2 | 5 | 5 | 5 | 5 | 5 |
| 78 | 30 | 0 | 3 | 1 | 5 | 5 | 5 | 5 | 4 | 0 | 5 | 5 | 5 |
| | 10 | 0 | 1 | 0 | 4 | 5 | 3 | 1 | 1 | 0 | 5 | 5 | 5 |
| 79 | 30 | 0 | 3 | 5 | 5 | 5 | 5 | 3 | 5 | 5 | 5 | 5 | 5 |
| | 10 | 0 | 2 | 3 | 5 | 5 | 5 | 0 | 2 | 5 | 5 | 5 | 5 |
| 80 | 30 | 0 | 2 | 2 | 3 | 2 | 5 | 0 | 1 | 5 | 5 | 5 | 5 |
| | 10 | 0 | 1 | 1 | 3 | 1 | 5 | 2 | 1 | 3 | 5 | 1 | 5 |

| Compound Nos. | Amount (g/a) | Pre-emergence | | | | | | Post-emergence | | | | | |
|---|---|---|---|---|---|---|---|---|---|---|---|---|---|
| | | A | B | C | D | E | F | A | B | C | D | E | F |
| 82 | 30 | 1 | 1 | 2 | 5 | 5 | 5 | 1 | 1 | 1 | 5 | 5 | 5 |
| | 10 | 0 | 0 | 2 | 1 | 2 | 5 | 0 | 0 | 1 | 3 | 1 | 5 |
| 83 | 30 | 0 | 0 | 2 | 2 | 2 | 4 | 0 | 1 | 1 | 1 | 2 | 0 |
| | 10 | 1 | 1 | 1 | 5 | 4 | 5 | 0 | 1 | 1 | 1 | 1 | 0 |
| 84 | 30 | 4 | 5 | 5 | 5 | 5 | 5 | 3 | 5 | 5 | 5 | 5 | 5 |
| | 10 | 1 | 5 | 5 | 5 | 4 | 5 | 1 | 2 | 5 | 5 | 5 | 5 |
| 86 | 30 | 0 | 1 | 1 | 0 | 0 | 3 | 0 | 1 | 5 | 4 | 5 | 5 |
| | 10 | 0 | 0 | 0 | 0 | 0 | 4 | 0 | 1 | 5 | 3 | 5 | 5 |
| 88 | 30 | 3 | 5 | 5 | 5 | 5 | 5 | 1 | 5 | 5 | 5 | 5 | 5 |
| | 10 | 0 | 2 | 3 | 5 | 5 | 5 | 0 | 5 | 5 | 5 | 5 | 5 |
| 103 | 30 | 0 | 5 | 5 | 5 | 5 | 5 | 1 | 5 | 5 | 5 | 5 | 5 |
| | 10 | 1 | 5 | 5 | 4 | 5 | 5 | 0 | 2 | 5 | 5 | 5 | 5 |
| 104 | 30 | 5 | 5 | 5 | 5 | 5 | 5 | 3 | 5 | 5 | 5 | 5 | 5 |
| | 10 | 0 | 5 | 5 | 5 | 5 | 5 | 0 | 3 | 4 | 5 | 5 | 1 |
| 105 | 30 | 0 | 5 | 5 | 5 | 5 | 5 | 5 | 5 | 5 | 5 | 5 | 5 |
| | 10 | 1 | 5 | 5 | 5 | 5 | 5 | 0 | 3 | 5 | 5 | 5 | 5 |
| 106 | 30 | 3 | 5 | 4 | 5 | 5 | 5 | 0 | 5 | 5 | 5 | 5 | 5 |
| | 10 | 1 | 3 | 3 | 5 | 5 | 5 | 0 | 3 | 5 | 5 | 5 | 5 |
| 107 | 30 | 5 | 5 | 5 | 5 | 5 | 5 | [illegible] | 5 | 5 | 5 | 5 | 5 |
| | 10 | 4 | 5 | 5 | 5 | 5 | 5 | 0 | 5 | 5 | 5 | 5 | 5 |

| Compound Nos. | Amount (g/a) | Pre-emergence | | | | | | Post-emergence | | | | | |
|---|---|---|---|---|---|---|---|---|---|---|---|---|---|
| | | A | B | C | D | E | F | A | B | C | D | E | F |
| 108 | 30 | 1 | 5 | 5 | 5 | 5 | 5 | 1 | 5 | 5 | 5 | 5 | 5 |
| | 10 | 0 | 5 | 3 | 3 | 3 | 4 | 1 | 5 | 5 | 5 | 5 | 5 |
| 109 | 30 | 0 | 5 | 5 | 5 | 5 | 5 | 2 | 5 | 5 | 5 | 5 | 5 |
| | 10 | 0 | 3 | 1 | 5 | 4 | 4 | 0 | 4 | 4 | 5 | 5 | 5 |
| 110 | 30 | 3 | 5 | 5 | 5 | 5 | 5 | 5 | 5 | 5 | 5 | 5 | 5 |
| | 10 | 0 | 5 | 4 | 5 | 5 | 5 | 2 | 5 | 5 | 5 | 5 | 5 |
| 111 | 30 | 2 | 5 | 5 | 5 | 5 | 5 | 2 | 5 | 5 | 5 | 5 | 5 |
| | 10 | 1 | 5 | 4 | 5 | 5 | 5 | 1 | 0 | 5 | 5 | 5 | 5 |
| 112 | 30 | 1 | 5 | 3 | 5 | 5 | 5 | 1 | 5 | 2 | 5 | 5 | 5 |
| | 10 | 0 | 2 | 0 | 5 | 5 | 5 | 0 | 3 | 3 | 5 | 5 | 5 |
| 113 | 30 | 0 | 0 | 1 | 2 | 2 | 2 | 0 | 1 | 5 | 5 | 5 | 5 |
| | 10 | 0 | 0 | 0 | 0 | 0 | 0 | 0 | 1 | 4 | 5 | 4 | 5 |
| 114 | 30 | 0 | 5 | 5 | 5 | 5 | 5 | 1 | 5 | 5 | 5 | 5 | 5 |
| | 10 | 0 | 5 | 5 | 5 | 5 | 5 | 1 | 5 | 5 | 5 | 5 | 5 |
| 115 | 30 | 0 | 2 | 2 | 1 | 3 | 3 | 0 | 0 | 2 | 5 | 4 | 5 |
| | 10 | 0 | 0 | 0 | 0 | 0 | 2 | 0 | 0 | 0 | 5 | 4 | 5 |
| 116 | 30 | 0 | 0 | 0 | 4 | 3 | 5 | 0 | 2 | 2 | 5 | 5 | 5 |
| | 10 | 0 | 0 | 0 | 2 | 1 | 5 | 0 | 1 | 1 | 5 | 5 | 5 |
| 117 | 30 | 0 | 0 | 1 | 3 | 3 | 4 | 0 | 1 | 5 | 5 | 5 | 5 |
| | 10 | 0 | 0 | 0 | 0 | 0 | 1 | 0 | 0 | 3 | 5 | 5 | 5 |

| Compound Nos. | Amount (g/a) | Pro-emergence | | | | | | Post-emergence | | | | | |
|---|---|---|---|---|---|---|---|---|---|---|---|---|---|
| | | A | B | C | D | E | F | A | B | C | D | E | F |
| 118 | 30 | 3 | 5 | 5 | 5 | 5 | 5 | 1 | 4 | 4 | 5 | 5 | 5 |
| | 10 | 1 | 5 | 1 | 5 | 5 | 5 | 1 | 1 | 4 | 5 | 5 | 5 |
| 119 | 30 | 1 | 5 | 5 | 5 | 4 | 5 | 1 | 4 | 5 | 5 | 5 | 5 |
| | 10 | 0 | 4 | 4 | 5 | 5 | 5 | 0 | 1 | 5 | 5 | 5 | 5 |
| 120 | 30 | 0 | 2 | 4 | 5 | 5 | 5 | 0 | 3 | 5 | 5 | 5 | 3 |
| | 10 | 0 | 1 | 3 | 5 | 5 | 5 | 0 | 2 | 5 | 1 | 1 | 2 |
| 121 | 30 | 0 | 1 | 1 | 5 | 4 | 5 | 1 | 1 | 3 | 5 | 5 | 5 |
| | 10 | 0 | 0 | 1 | 1 | 1 | 5 | 0 | 0 | 1 | 5 | 4 | 5 |
| 122 | 30 | 1 | 5 | 5 | 5 | 3 | 5 | 1 | 3 | 5 | 5 | 4 | 5 |
| | 10 | 0 | 3 | 5 | 4 | 1 | 5 | 0 | 1 | 5 | 5 | 4 | 5 |
| 123 | 30 | 0 | 2 | 1 | 5 | 5 | 5 | 1 | 1 | 4 | 5 | 5 | 5 |
| | 10 | 0 | 1 | 1 | 4 | 2 | 5 | 1 | 1 | 2 | 5 | 4 | 5 |
| 124 | 30 | 1 | 2 | 3 | 4 | 5 | 5 | 1 | 3 | 1 | 5 | 5 | 5 |
| | 10 | 0 | 0 | 1 | 1 | 0 | 3 | 0 | 1 | 0 | 5 | 5 | 5 |
| 125 | 30 | 1 | 1 | 3 | 5 | 2 | 5 | 0 | 2 | 3 | 5 | 1 | 2 |
| | 10 | 0 | 0 | 0 | 0 | 0 | 4 | 0 | 1 | 2 | 3 | 0 | 1 |
| 126 | 30 | 0 | 1 | 0 | 1 | 2 | 0 | 0 | 1 | 0 | 3 | 5 | 3 |
| | 10 | 0 | 0 | 0 | 0 | 1 | 1 | 0 | 0 | 1 | 0 | 5 | 2 |
| Reference compound 1 | 30 | 0 | 4 | 5 | 5 | 3 | 5 | 0 | 2 | 2 | 1 | 2 | 4 |
| | 10 | 0 | 2 | 4 | 5 | 1 | 5 | 0 | 1 | 2 | 1 | 1 | 4 |

The every compound of the present invention has an excellent herbicidal activity both in the pre-emergence test and in the post-emergence test.

Experiment 2

a) Test Method (Pre-emergence test)

Seeds of a test compound were sowed on the loam charged in a square plastic-pot (10 x 10 cm). After sowing, the seeds were covered with sandy loam and an aqueous suspension (Tween 20 was used at a concentration of 100 ppm as a spreader.) of the test compound was applied over the surface of the sandy loam.

The suspension of the test compound was prepared by dilution with water at a concentration of 20g/10L, 10g/10L, 5g/10L or 2.5g/10L and applied at a rate of 10L/are with an automatic metal sprayer.

The pots were kept at a temperature of 25°C in a greenhouse under the natural sunlight.

b) Method of evaluation

The outward change of the seeds or plants was checked by eye observation at prefixed intervals , and after four weeks the evaluation was made on the following six degrees according to the degree of damages to the plant.

| Degree of Damages | Score |
|---|---|
| complete death | 5 |
| severe | 4 |
| moderate | 3 |
| mild | 2 |
| slight | 1 |
| none | 0 |

c) Results

Results in the test are summarized in Table 16.

Table 16

| Compound Nos. | Amount (g/a) | Weeds | | | | Crop plants | | | | | | |
|---|---|---|---|---|---|---|---|---|---|---|---|---|
| | | large crabgrass | barnyard grass | smartweed | spleen amaranth | corn | wheat | soybean | cotton | sugar beet | oilseed rape | tomato |
| 4 | 20 | 5 | 2 | 5 | 5 | 0 | 1 | 5 | 0 | 5 | 4 | 0 |
| | 10 | 3 | 2 | 5 | 5 | 0 | 0 | 4 | 0 | 5 | 4 | 0 |
| | 5 | 2 | 0 | 3 | 5 | 0 | 0 | 2 | 0 | 5 | 3 | 0 |
| | 2.5 | 1 | 0 | 3 | 5 | 0 | 0 | 0 | 0 | 3 | 2 | 0 |
| 49 | 20 | 5 | 5 | 5 | 5 | 2 | 5 | 3 | 5 | 5 | 5 | 5 |
| | 10 | 5 | 4 | 5 | 5 | 1 | 4 | 3 | 4 | 5 | 5 | 4 |
| | 5 | 5 | 2 | 5 | 5 | 1 | 3 | 2 | 2 | 5 | 3 | 3 |
| | 2.5 | 3 | 2 | 4 | 4 | 0 | 2 | 1 | 0 | 4 | 0 | 2 |
| 61 | 20 | 5 | 5 | 5 | 5 | 0 | 0 | 0 | 4 | 5 | 5 | 4 |
| | 10 | 3 | 3 | 5 | 5 | 0 | 0 | 0 | 2 | 5 | 5 | 3 |
| | 5 | 3 | 3 | 3 | 5 | 0 | 0 | 0 | 2 | 5 | 3 | 2 |
| | 2.5 | 3 | 3 | 3 | 5 | 0 | 0 | 0 | 1 | 4 | 3 | 1 |
| 62 | 20 | 5 | 4 | 5 | 5 | 3 | 5 | 4 | 5 | 5 | 5 | 5 |
| | 10 | 4 | 3 | 5 | 5 | 0 | 4 | 2 | 3 | 5 | 4 | 4 |
| | 5 | 2 | 2 | 5 | 5 | 0 | 2 | 2 | 2 | 5 | 4 | 2 |
| | 2.5 | 0 | 0 | 0 | 3 | 0 | 0 | 1 | 2 | 5 | 3 | 1 |

| Compound Nos. | Amount (g/a) | Weeds | | | | Crop plants | | | | | | |
|---|---|---|---|---|---|---|---|---|---|---|---|---|
| | | large crabgrass | barnyard grass | smartweed | spleen amaranth | corn | wheat | soybean | cotton | sugar beet | oilseed rape | tomato |
| 64 | 20 | 5 | 5 | 5 | 5 | 0 | 4 | 3 | 5 | 5 | 5 | 5 |
| | 10 | 5 | 5 | 5 | 5 | 0 | 4 | 2 | 3 | 5 | 5 | 4 |
| | 5 | 2 | 2 | 3 | 5 | 0 | 0 | 0 | 2 | 5 | 3 | 2 |
| | 2.5 | 0 | 0 | 2 | 5 | 0 | 0 | 0 | 2 | 3 | 2 | 2 |
| 66 | 20 | 5 | 5 | 5 | 5 | 2 | 3 | 3 | 4 | 5 | 5 | 5 |
| | 10 | 5 | 5 | 5 | 5 | 1 | 2 | 0 | 3 | 5 | 5 | 4 |
| | 5 | 5 | 4 | 5 | 5 | 0 | 0 | 0 | 2 | 5 | 5 | 3 |
| | 2.5 | 4 | 3 | 4 | 5 | 0 | 0 | 0 | 0 | 5 | 3 | 2 |
| 73 | 20 | 5 | 5 | 5 | 5 | 4 | 5 | 5 | 5 | 5 | 5 | 5 |
| | 10 | 5 | 5 | 5 | 5 | 0 | 3 | 0 | 3 | 5 | 5 | 5 |
| | 5 | 5 | 4 | 5 | 5 | 0 | 2 | 0 | 2 | 5 | 5 | 2 |
| | 2.5 | 3 | 2 | 4 | 5 | 0 | 0 | 0 | 1 | 4 | 2 | 2 |
| 76 | 20 | 4 | 4 | 5 | 5 | 1 | 0 | 2 | 4 | 5 | 5 | 4 |
| | 10 | 3 | 4 | 5 | 5 | 0 | 0 | 1 | 2 | 5 | 5 | 2 |
| | 5 | 2 | 2 | 5 | 5 | 0 | 0 | 0 | 0 | 4 | 3 | 0 |
| | 2.5 | 0 | 0 | 0 | 0 | 0 | 0 | 0 | 0 | 3 | 0 | 0 |
| 79 | 20 | 5 | 5 | 5 | 5 | 3 | 5 | 4 | 5 | 5 | 5 | 5 |
| | 10 | 5 | 5 | 5 | 5 | 2 | 4 | 3 | 4 | 5 | 5 | 3 |
| | 5 | 5 | 4 | 5 | 5 | 0 | 3 | 0 | 2 | 5 | 4 | 3 |
| | 2.5 | 3 | 0 | 3 | 5 | 0 | 0 | 0 | 0 | 5 | 3 | 2 |

| Compound Nos. | Amount (g/a) | Weeds: large crabgrass | barnyard grass | smartweed | spleen amaranth | Crop plants: corn | wheat | soybean | cotton | sugar beet | oilseed rape | tomato |
|---|---|---|---|---|---|---|---|---|---|---|---|---|
| Reference compound 2 | 20 | 5 | 5 | 5 | 5 | 2 | 4 | 2 | 3 | 5 | 5 | 5 |
| | 10 | 5 | 5 | 5 | 5 | 1 | 3 | 2 | 2 | 5 | 5 | 4 |
| | 5 | 3 | 2 | 3 | 3 | 0 | 1 | 0 | 0 | 4 | 3 | 3 |
| | 2.5 | 2 | 1 | 2 | 2 | 0 | 0 | 0 | 0 | 3 | 2 | 0 |

The test-compounds each showed an excellent herbicidal activity against large crabgrass, barnyard grass, smartweed and spleen amaranth.

Especially, no damages were observed in the tests with the compounds Nos. 4, 61 and 64 for corn, the compound No. 61 for wheat and soybean, and the compound No.4 for cotton and tomato, so they are very hopeful selective herbicides applicable to the field of the above-specified crop plants, respectively. Additionally, the compounds other than the above specified are also useful as non-selective herbicides.

The formulations of the herbicides of the present invention are illustrated as follows.

Formulation 1

Ten parts by weight of 3-(3-t-butyl-5-isoxazolyl)-1-methyl-2-imidazolidinone and 90 parts by weight of dust talc are mixed up uniformly to give a dust.

Formulation 2

Twenty parts by weight of 1-methyl-3-(5-isopropyl-3-isoxazolyl)-4-methoxycarbonyloxy-2-imidazolidinone, 50 parts by weight of clay, 25 parts by weight of talc and 5 parts by weight of Emal (Registered trademark, Kao Soap Co.) as a spreader are mixed to give a wettable powder.

Formulation 3

To a solution of 20 parts by weight of 3-(3-t-butyl-5-isoxazolyl)-1-methyl-5-methoxycarbonyloxy-2-imidazolidinone in 65 parts by weight of dimethylformamide are added 15 parts by weight of Sorpol (Registered trademark, Toho Chemical Ind.) to give an emulsion.

What we claim is:

1. A compound of the formula:

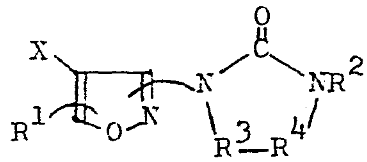

(wherein $R^1$ is $C_1$- $C_5$ alkyl; $R^2$ is hydrogen, $C_1$- $C_5$ alkyl, or $C_2$- $C_5$ alkenyl; $-R^3-R^4-$ is $-\underset{R^5}{CH}-(CH_2)_n-$, $-(CH_2)_n-\underset{R^5}{CH}-$,

$-CO-CH_2-$, -CO-CO-, -CH(OY)-CH(OY)-. $-CH(OY)-CH_2-$, 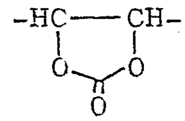

-CH(OZ)-CO-, or -CO-CH(OZ)-; $R^5$ is hydrogen or $C_1$- $C_3$ alkyl; n is 1 or 2: X is hydrogen or halogen; Y is $C_1$- $C_5$ alkyl, $C_2$- $C_5$ alkenyl, $C_2$- $C_5$ alkoxycarbonyl, or N-($C_1$- $C_3$ alkyl)-carbamoyl; and Z is hydrogen, $C_2$- $C_5$ alkanoyl, $C_2$- $C_5$ alkoxycarbonyl, N-($C_1$- $C_3$ alkyl)carbamoyl, or benzoyl which may be substituted).

2. A compound claimed in Claim 1, namely 3-(5-tert-butyl-4-chloro-3-isoxazolyl)-1-methyl-2-imidazolidinone.

3. A compound claimed in Claim 1, namely 3-(3-tert-butyl-5-isoxazolyl)-1-methyl-2-imidazolidinone.

4. A compound claimed in Claim 1, namely 3-(5-isopropyl-3-isoxazolyl)-1-methyl-4-methoxycarbonyloxy-2-imidazolidinone.

5. A compound claimed in Claim 1, namely 3-(3-tert-butyl-5-isoxazolyl)-1-methyl-4-methoxycarbonyloxy-2-imidazolidinone.

6. A compound claimed in Claim 1, namely 3-(5-tert-butyl-3-isoxazolyl)-1-methyl-5-methoxycarbonyloxy-2,4-dioxo-imidazolidine.

7. A compound claimed in Claim 1, namely 3-(5-tert-butyl-3-isoxazolyl)-1-methyl-4-(N-methylcarbamoyloxy)-2-imidazolidinone.

8. A compound claimed in Claim 1, namelyl 3-(5-isopropyl-3-isoxazolyl)-1-methyl-4-(N-methylcarbamoyloxy)-2-imidazolidinone.

9. A herbicidal composition containing one or more species of compounds claimed in Claim 1 as an effective ingredient together with agricultural carriers, diluents, and/or excipients.

European Patent Office

**EUROPEAN SEARCH REPORT**

0144098
Application number

EP 84 20 1498

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| A | US-A-4 354 030 (K.W.BUROW et al.)<br>--- | | C 07 D 413/04<br>C 07 D 498/04<br>A 01 N 43/50<br>A 01 N 43/80 |
| A | EP-A-0 044 185 (ELI LILLY & CO.)<br>----- | | |
| | | | TECHNICAL FIELDS SEARCHED (Int. Cl 4) |
| | | | C 07 D 413/00<br>C 07 D 498/00<br>A 01 N 43/00 |

The present search report has been drawn up for all claims

| Place of search | Date of completion of the search | Examiner |
|---|---|---|
| THE HAGUE | 30-01-1985 | DE BUYSER I. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82